# EUROPEAN PATENT APPLICATION

(11) **EP 4 023 297 A1**
(43) Date of publication of application: **06.07.2022**
(21) Application number: 20856670.3
(22) Date of filing: 27.08.2020
(51) Int. Cl.: A61P 35/00, A61P 35/04, A61K 47/56, C12N 15/113, A61K 31/7088

(54) **NUCLEIC ACID MEDICINE FOR TARGETING GASTRIC CANCER MOLECULE**

(30) Priority: 27.08.2019 JP 2019154968
(71) Applicant: National University Corporation Tokai National Higher Education and Research System, Nagoya-shi, Aichi 464-8601 (JP); National Institutes of Biomedical Innovation, Health and Nutrition, Ibaraki-shi, Osaka 567-0085 (JP)
(72) Inventor: KANDA Mitsuro, Nagoya-shi, Aichi 464-8601 (JP); OBIKA Satoshi, Ibaraki-shi, Osaka 567-0085 (JP); KASAHARA Yuya, Ibaraki-shi, Osaka 567-0085 (JP)
(74) Representative: Wohlfahrt, Jan Günther
(86) International application number: PCT/JP2020/032270
(87) International publication number: WO 2021/039879

(57) **Abstract**

A nucleic acid medicine using an antisense nucleic acid to inhibit the expression of SYT13, which has better efficacy than siRNA for targeting peritoneal dissemination of gastric cancer is provided. An antisense oligonucleotide which consists of a nucleotide sequence substantially complementary to the nucleotide sequence of a specific region in human SYT13 mRNA and which can inhibit the expression of human SYT13 mRNA; and a pharmaceutical composition comprising the antisense oligonucleotide are also provided.

## Description

### Technical Field

The present invention relates to a molecular targeted nucleic acid medicine for targeting gastric cancer, particularly peritoneal dissemination of gastric cancer, and more specifically to an antisense oligonucleotide against SYT13 and a pharmaceutical composition comprising the same.

### Background Art

Gastric cancer is common in Asia, including Japan, China, and Korea, as well as in South America. Although the spread of cancer screening has enabled early detection and treatment, and mortality from gastric cancer has decreased, advanced gastric cancer still has a poor prognosis and is an important disease to be overcome.

Peritoneal dissemination is known as one of the recurrence and metastasis forms of gastric cancer. Peritoneal dissemination is most common in cases with stage IV at diagnosis and is also the most common form of recurrence after resection. Further, peritoneal dissemination has a major problem that the efficacy of treatment with resection, radiotherapy, and systemic administration of anticancer drugs is low.

The present inventors' group has previously found that the expression of SYT13 is upregulated specifically in gastric cancer causing peritoneal dissemination, and has reported that the expression can be used as an indicator to forecast peritoneal dissemination after gastric resection and that siRNA against SYT13 can inhibit the proliferative capacity, invasive capacity, and migration capacity of a gastric cancer cell line and can inhibit metastasis through peritoneal dissemination after gastric resection (Patent Literature 1 and Non Patent Literature 1).

SYT13 is a membrane protein belonging to the synaptotagmin (SYT) family. SYT family proteins have been identified as calcium-phospholipid binding molecules on synaptic vesicles and have been suggested to function as a calcium sensor. It has been reported that humans have 17 isoforms distributed mainly in brain tissue. It has also been reported that SYT13, unlike other synaptotagmins, binds to phospholipids regardless of the presence or absence of calcium and is also expressed in various tissues other than the brain (Non Patent Literature 2).

### Citation List

### Patent Literature

Patent Literature 1: International Publication No. WO2016/143697

### Non Patent Literature

Non Patent Literature 1: M. Kanda et al., British Journal of Surgery, 2018; 108: 1349-1358
Non Patent Literature 2: M. Fukuda and K. Mikoshiba, Biochem J., 2001; 354: 249-257

### Summary of Invention

### Technical Problem

As described above, it has been confirmed that the use of siRNA against SYT13 can inhibit the expression of SYT13 and metastasis through peritoneal dissemination; however, the sequence information on the siRNA actually used is unpublished, and the inhibition of SYT13 expression has not been fully investigated.

Thus, the present inventors have now made an object to provide a nucleic acid medicine having better efficacy for targeting peritoneal dissemination of gastric cancer, by using an antisense oligonucleotide, which is a nucleic acid medicine different from siRNA, to inhibit the expression of SYT13.

### Solution to Problem

In view of the above-mentioned object, the present inventors first focused on the nucleotide sequence of human SYT13 mRNA; as a result of repeated investigations to obtain an antisense oligonucleotide with effectiveness as a medicine and without the possibility of adverse effects, they found that targeting a specific region in the nucleotide sequence of SYT13 mRNA attains particularly high efficacy, leading to the completion of the present invention.

That is, the present invention provides the followings:
1. An antisense oligonucleotide capable of inhibiting expression of human SYT13 mRNA, the antisense oligonucleotide being:
   an antisense oligonucleotide consisting of a 11- to 19-base-long nucleotide sequence complementary to a nucleotide sequence at positions 348 to 366, 599 to 627, 997 to 1016, 1069 to 1088, 1419 to 1437, 1612 to 1641, 1775 to 1793, 2629 to 2647, 2810 to 2831, 3244 to 3262, 3315 to 3333, 3423 to 3442, 4266 to 4284, 4328 to 4346, or 4365 to 4400, 4714 to 4751, 4776 to 4795, 4949 to 4968 in a nucleotide sequence set forth in SEQ ID NO: 1; or
   an antisense oligonucleotide consisting of a nucleotide sequence in which one to three bases, one to two bases, or one base are substituted, deleted, or inserted with regard to said antisense oligonucleotide.
2. An antisense oligonucleotide consisting of a nucleotide sequence selected from the group consisting of SEQ ID NOs: 3 to 43, or an antisense oligonucleotide consisting of a nucleotide sequence in which one to three bases are substituted, deleted, or inserted with regard to said antisense oligonucleotide.
3. An antisense oligonucleotide consisting of a nucleotide sequence selected from the group consisting of SEQ ID NOs: 50 to 59, or an antisense oligonucleotide consisting of a nucleotide sequence in which one to three bases are substituted, deleted, or inserted with regard to said antisense oligonucleotide.
4. An antisense oligonucleotide consisting of a nucleotide sequence selected from the group consisting of SEQ ID NOs: 60 to 69, or an antisense oligonucleotide consisting of a nucleotide sequence in which one to three bases are substituted, deleted, or inserted with regard to said antisense oligonucleotide.
5. An antisense oligonucleotide consisting of a nucleotide sequence selected from the group consisting of SEQ ID NOs: 70 to 79, or an antisense oligonucleotide consisting of a nucleotide sequence in which one to three bases are substituted, deleted, or inserted with regard to said antisense oligonucleotide.
6. An antisense oligonucleotide consisting of a nucleotide sequence selected from the group consisting of SEQ ID NOs: 4, 20, 29, 35, 39, 62, and 79, or an antisense oligonucleotide consisting of a nucleotide sequence in which one to three bases are substituted, deleted, or inserted with regard to said antisense oligonucleotide.
7. The antisense oligonucleotide according to any one of 1 to 6 above, wherein the antisense oligonucleotide has an artificial nucleic acid region containing a bicyclic sugar.
8. The antisense oligonucleotide according to any one of 1 to 7 above, wherein at least one internucleoside linkage is a phosphorothioate bond.
9. The antisense oligonucleotide according to any one of 1 to 8 above, wherein the antisense oligonucleotide has an artificial nucleic acid region containing 5-methylcytosine.
10. The antisense oligonucleotide according to any one of 1 to 9 above, wherein the antisense oligonucleotide is 15 to 19 nucleotides in length.
11. The antisense oligonucleotide according to any one of 1 to 10 above, wherein the antisense oligonucleotide is a gapmer.
12. A conjugate comprising: the antisense oligonucleotide according to any one of 1 to 11 above; and a further functional moiety directly or indirectly linked to the antisense oligonucleotide.
13. The conjugate according to 12 above, wherein the further functional moiety is a ligand for target molecule or an agent having antitumor activity.
14. A pharmaceutical composition comprising the antisense oligonucleotide according to any one of 1 to 11 above, or the conjugate according to 12 or 13 above.
15. The pharmaceutical composition according to 14 above, for treatment or prevention of gastric cancer in a human.
16. The pharmaceutical composition according to 15 above, for treatment or prevention of peritoneal dissemination after gastric cancer resection.

The present specification includes the disclosure of Japanese Patent Application No. 2019-154968, which is the basis of the priority of the present application.

### Advantageous Effects of Invention

The present invention can provide a molecular targeted nucleic acid medicine for gastric cancer that is delivered specifically to a region where SYT13 is highly expressed to inhibit the expression and thus is significantly superior to siRNA.

### Brief Description of Drawings

[Figure 1] Figure 1 schematically shows exemplary structures of an antisense nucleic acid that can be used in the present invention.
[Figure 2] Figure 2 shows that the antisense nucleic acid of the present invention inhibits the expression of SYT13 in KATOIII cells in concentration-dependent manner. Control: no antisense nucleic acids added; NEG1: a control antisense nucleic acid added.
[Figure 3] Figure 3 shows that the antisense nucleic acid of the present invention inhibits the expression of SYT13 in MKN1 cells in concentration-dependent manner. Control: no antisense nucleic acids added; NEG1: a control antisense nucleic acid added.
[Figure 4] Figure 4 shows that the antisense nucleic acid of the present invention inhibits the expression of SYT13 in MKN45 cells in concentration-dependent manner. Control: no antisense nucleic acids added; NEG2: a control antisense nucleic acid added.
[Figure 5] Figure 5 shows that the antisense nucleic acid of the present invention inhibits the expression of SYT13 in OCUM-1 cells in concentration-dependent manner. Control: no antisense nucleic acids added; NEG2: a control antisense nucleic acid added.
[Figure 6] Figure 6 shows that the antisense nucleic acid of the present invention inhibits the expression of SYT13 in OCUM-1 cells in concentration-dependent manner. Control: no antisense nucleic acids added; NEG2: a control antisense nucleic acid added.
[Figure 7] Figure 7 shows that the antisense nucleic acid of the present invention inhibits the expression of SYT13 in MKN1 cells in concentration-dependent manner. A dotted line and a dashed line indicate expression inhibition levels with the use of 100nM and 400nM of parental sequence hSYT13-4729-AmNA (15), respectively. Control: no antisense nucleic acids added; NEG2: a control antisense nucleic acid added.
[Figure 8] Figure 8 shows that the antisense nucleic acid of the present invention inhibits the expression of SYT13 in NUGC-4 cells in concentration-dependent manner. A dotted line and a dashed line indicate expression inhibition levels with the use of 100nM and 400nM of parental sequence hSYT13-4378-AmNA (15), respectively. Control: no antisense nucleic acids added; NEG1 and NEG2: a control antisense nucleic acid added.
[Figure 9] Figure 9 shows that the antisense nucleic acid of the present invention inhibits the expression of SYT13 in NUGC-4 cells in concentration-dependent manner. A dotted line and a dashed line indicate expression inhibition levels with the use of 100nM and 400nM of parental sequence hSYT13-4729-AmNA (15), respectively. Control: no antisense nucleic acids added; NEG1 and NEG2: a control antisense nucleic acid added.
[Figure 10A] Figure 10A shows an effect of the antisense nucleic acid of the present invention on the proliferative capacity of MKN1 cells. Con: no antisense nucleic acids added; NEG1: a control antisense nucleic acid added.
[Figure 10B] Figure 10B shows an effect of the antisense nucleic acid of the present invention on the proliferative capacity of KATO-III cells. Con: no antisense nucleic acids added; NEG1: a control antisense nucleic acid added.
[Figure 10C] Figure 10C shows an effect of the antisense nucleic acid of the present invention on the proliferative capacity of OCUM-1 cells. Con: no antisense nucleic acids added; NEG1: a control antisense nucleic acid added.
[Figure 10D] Figure 10D shows an effect of the antisense nucleic acid of the present invention on the proliferative capacity of AGS cells. Con: no antisense nucleic acids added; NEG1: a control antisense nucleic acid added.
[Figure 10E] Figure 10E shows an effect of the antisense nucleic acid of the present invention on the proliferative capacity of N87 cells. Con: no antisense nucleic acids added; NEG1: a control antisense nucleic acid added.
[Figure 10F] Figure 10F shows an effect of the antisense nucleic acid of the present invention on the proliferative capacity of NUGC4 cells. Con: no antisense nucleic acids added; NEG1: a control antisense nucleic acid added.
[Figure 10G] Figure 10G shows an effect of the antisense nucleic acid of the present invention on the proliferative capacity of GSU cells. Con: no antisense nucleic acids added; NEG1: a control antisense nucleic acid added.
[Figure 11A] Figure 11A shows an effect of siRNA on the proliferative capacity of MKN1 cells. Control: no siRNA added; siControl: control siRNA added.
[Figure 11B] Figure 11B shows an effect of siRNA on the proliferative capacity of NUGC4 cells. Control: no siRNA added; siControl: control siRNA added.
[Figure 12A] Figure 12A shows an effect of the antisense nucleic acid of the present invention on the migration capacity of MKN1/Luc cells. The antisense nucleic acid at a final concentration of 400 nM was added to MKN1/Luc cells (3 × 10⁴ cells/well), the cells were photographed at 20 evenly-spaced locations every 6 hours up to 18 hours and measured with Image-J, and the mean value and standard deviation of these measurements were calculated. Control: no antisense nucleic acids added; NEG1: a control antisense nucleic acid added.
[Figure 12B] Figure 12B shows an effect of the antisense nucleic acid of the present invention on the migration capacity of N87 cells. The antisense nucleic acid at a final concentration of 100 nM was added to N87 cells (30 × 10⁴ cells/well), the cells were photographed at 20 evenly-spaced locations every 12 hours up to 60 hours and measured with Image-J, and the mean value and standard deviation of these measurements were calculated. Control: no antisense nucleic acids added; NEG1: a control antisense nucleic acid added.
[Figure 12C] Figure 12C shows an effect of the antisense nucleic acid of the present invention on the migration capacity of NUGC4 cells. The antisense nucleic acid at a final concentration of 100 nM was added to NUGC4 cells (3.5 × 10⁴ cells/well), the cells were photographed at 20 evenly-spaced locations every 6 hours up to 24 hours and measured with Image-J, and the mean value and standard deviation of these measurements were calculated. Control: no antisense nucleic acids added; NEG1: a control antisense nucleic acid added.
[Figure 13A] Figure 13A shows an effect of the antisense nucleic acid of the present invention on the invasive capacity of MKN1 cells. The antisense nucleic acid at a final concentration of 400 nM was added to MKN1/Luc cells (2.5 × 10⁴ cells/well), the cells were collected 36 hours after seeding; afterward, the number of cells were counted in 8 sections for each of selected 4 fields of view, i.e., in 32 areas in total, and the mean value and standard deviation of the resultant cell counts were calculated. Control: no antisense nucleic acids added; NEG1: a control antisense nucleic acid added.
[Figure 13B] Figure 13B shows an effect of the antisense nucleic acid of the present invention on the invasive capacity of AGS cells. The antisense nucleic acid at a final concentration of 100 nM was added to AGS cells (5 × 10⁴ cells/well), the cells were collected 48 hours after seeding; afterward, the number of cells were counted in 8 sections for each of selected 4 fields of view, i.e., in 32 areas in total, and the mean value and standard deviation of the resultant cell counts were calculated. Control: no antisense nucleic acids added; NEG1: a control antisense nucleic acid added.
[Figure 13C] Figure 13C shows an effect of the antisense nucleic acid of the present invention on the invasive capacity of GSU cells. The antisense nucleic acid at a final concentration of 100 nM was added to GSU cells (5 × 10⁴ cells/well), the cells were collected 144 hours after seeding; afterward, the number of cells were counted in 8 sections for each of selected 4 fields of view, i.e., in 32 areas in total, and the mean value and standard deviation of the resultant cell counts were calculated. Control: no antisense nucleic acids added; NEG1: a control antisense nucleic acid added.
[Figure 14A] Figure 14A shows an overview of an *in vivo* test on efficacy of the antisense nucleic acid of the present invention using a mouse model.
[Figure 14B] Figure 14B shows efficacy of the antisense nucleic acid of the present invention on total weight of peritoneal dissemination lesion in an MKN1-administered mouse. Control: no antisense nucleic acids administration; NEG1: a control antisense nucleic acid administration.
[Figure 14C] Figure 14C shows efficacy of the antisense nucleic acid of the present invention on total weight of peritoneal dissemination lesion in an NUGC4-administered mouse. Control: no antisense nucleic acids administration; NEG1: a control antisense nucleic acid administration.
[Figure 15A] Figure 15A shows an effect of the antisense oligonucleotide of the present invention on the proliferative capacity of MKN1 cells. Cont: no antisense oligonucleotides added; NEG1: a control antisense nucleic acid added.
[Figure 15B] Figure 15B shows an effect of the antisense oligonucleotide of the present invention on the proliferative capacity of NUGC4 cells. Cont: no antisense oligonucleotides added; NEG1: a control antisense nucleic acid added.
[Figure 16A] Figure 16A shows an effect of the antisense oligonucleotide of the present invention on the migration capacity of MKN1 cells. Cont: no antisense oligonucleotides added; NEG1: a control antisense nucleic acid added.
[Figure 16B] Figure 16B shows an effect of the antisense oligonucleotide of the present invention on the migration capacity of NUGC4 cells. Cont: no antisense oligonucleotides added; NEG1: a control antisense nucleic acid added.
[Figure 17A] Figure 17A shows an effect of the antisense oligonucleotide of the present invention on the invasive capacity of MKN1 cells. Control: no antisense oligonucleotides added; NEG1: a control antisense nucleic acid added.
[Figure 17B] Figure 17B shows an effect of the antisense oligonucleotide of the present invention on the invasive capacity of MKN1 cells. Cont: no antisense oligonucleotide added; NEG1: a control antisense nucleic acid added.
[Figure 18A] Figure 18A shows an overview of an *in vivo* test on efficacy of the antisense oligonucleotide of the present invention using a mouse model.
[Figure 18B] Figure 18B shows efficacy of the antisense oligonucleotide of the present invention on total weight of peritoneal dissemination lesion in an NUGC4-administered mouse. Control: no antisense oligonucleotides administration; NEG1: a control antisense nucleic acid administration.
[Figure 18C] Figure 18C shows a result of a survival analysis with and without administration of the antisense oligonucleotide of the present invention. CEM: no antisense oligonucleotides administration; NEG1: a control antisense nucleic acid administration.
[Figure 19] Figure 19 shows that the antisense oligonucleotides with different modifications inhibit the expression of SYT13 in NUGC-4 cells in concentration-dependent manner. Control: no antisense oligonucleotides added; NEG1: a control antisense oligonucleotide added.

### Description of Embodiments

Hereinafter, the present invention will be described in detail.

### (Antisense oligonucleotide)

The present invention relates to an antisense oligonucleotide. More specifically, the present invention relates to an antisense oligonucleotide having a sequence substantially complementary to a part of the nucleotide sequence of SYT13 mRNA, the antisense oligonucleotide being capable of inhibiting the expression of human SYT13.

The SYT13 gene is present in mammals, such as primates (e.g., cynomolgus monkeys, chimpanzees, and humans) and non-primates (e.g., cattle, pigs, sheep, horses, cats, dogs, guinea pigs, rats, and mice); its nucleotide sequences and amino acid sequences of the SYT13 protein encoded by the nucleotide sequences can be obtained from databases such as a database of the National Center for Biotechnology Information (NCBI). It is also known that there are multiple isoforms for SYT13; examples of human SYT13 mRNA sequence include those set forth in SEQ ID NO: 1 (NM_020826.2) and SEQ ID NO:2 (NM_001247987.1) (shown as DNA sequences in SEQ ID NOs: 1 and 2).

The term "antisense oligonucleotide" as used herein is used synonymously with the term "antisense nucleic acid" commonly used in this field, and refers to a single-stranded oligonucleotide comprising a nucleobase sequence capable of hybridizing to (i.e., complementary to) a part of mRNA of the target gene, SYT13. Although not bound by theory, in the present invention, the antisense oligonucleotide forms a DNA-RNA hybrid with a target RNA, which upon being cleaved by RNaseH degrades the target RNA and thus can inhibit the expression of the target gene. In general, regions in the mRNA of a target gene where an antisense oligonucleotide can hybridize may include 3'UTR, 5'UTR, exons, introns, coding regions, a translation initiation region, a translation termination region, or other nucleic acid regions.

In the present invention, the antisense oligonucleotide can inhibit the expression of human SYT13 mRNA. More specifically, the antisense oligonucleotide of the present invention consists of a nucleotide sequence that is substantially complementary to the nucleotide sequence of a specific region of human SYT13 mRNA. Although not particularly limited, when an animal model transplanted with human cancer cells is used to examine the inhibition of the expression of human SYT13 mRNA, it is preferable to use an antisense oligonucleotide consisting of a nucleotide sequence that is not complementary to the nucleotide sequence of SYT13 mRNA of the animal (e.g., mouse) itself.

The term "inhibition" with respect to the expression of a gene as used herein refers to reduction of the amount (abundance) of mRNA created through transcription of the gene. The inhibition involves inhibiting the amount of mRNA by 20% or more, 30% or more, or 40% or more, preferably 50% or more, more preferably 80% or more, 90% or more, or 95% or more as compared to a control. The inhibition of gene expression may be determined by any method known in this technical field, but in particular, it may be determined by PCR-based methods such as real-time PCR using cells such as human or mouse cells.

The term "nucleobase" or "base" as used herein refers to a base component of a nucleic acid, which is the heterocycle moiety that can be paired with a base of another nucleic acid. The term "nucleobase sequence" as used herein may refer to a consecutive sequence of nucleobases, not taking into account the sugar, internucleoside linkage, or nucleobase modification that constitutes the nucleic acid.

In one embodiment, the antisense oligonucleotide may comprise a 11- to 19-base-long consecutive nucleobase sequence that are substantially complementary to human SYT13 mRNA, e.g., the nucleotide sequence set forth in SEQ ID NO:1 and/or SEQ ID NO:2. The consecutive nucleobase sequence may be 11 or more, 12 or more, 13 or more, 14 or more, 15 or more, 16 or more, 17 or more, 18 or more, or 19 bases long, e.g., 13, 15, 17, or 19 bases long. The antisense oligonucleotide may consist of a 11- to 19-base-long consecutive nucleobase sequence that are substantially complementary to human SYT13 mRNA, e.g., the nucleotide sequence set forth in SEQ ID NO:1 and/or SEQ ID NO:2.

The term "substantially complementary" refers to a nucleotide sequence that is completely complementary to a nucleotide sequence of interest as well as a nucleotide sequence that has one to several mismatches to a nucleotide sequence of interest but can form complementary base pairs therewith. That is, the antisense oligonucleotide may include a nucleobase sequence complementary to a nucleotide sequence of interest, e.g., a 11- to 19-base-long consecutive nucleotide sequence in which one to several nucleobases, e.g., one, two, or three nucleobases, are substituted, deleted, or inserted (in particular, substituted).

In the present invention, the nucleobase sequence of the antisense oligonucleotide may have no mismatch or 1 to 3, 1 to 2, or 1 mismatch to a part of human SYT13 mRNA. The term "a part" of mRNA refers to a target region in the mRNA where the antisense oligonucleotide can hybridize through base pairing, and the target region can have the same base length as the antisense oligonucleotide. The term "mismatch" refers to inability for a nucleobase of a first nucleic acid to form a base pair with (be complementary to) its corresponding nucleobase of a second nucleic acid.

In a preferred embodiment, the antisense oligonucleotide has a nucleotide sequence that is completely complementary to the nucleotide sequence set forth in SEQ ID NO:1 and/or SEQ ID NO:2.

More specifically, the present invention provides an antisense oligonucleotide capable of inhibiting the expression of human SYT13 mRNA, the antisense oligonucleotide being: an antisense oligonucleotide consisting of a 11- to 19-base-long nucleotide sequence complementary to a nucleotide sequence at positions 348 to 366, 599 to 627, 997 to 1016, 1069 to 1088, 1419 to 1437, 1612 to 1641, 1775 to 1793, 2629 to 2647, 2810 to 2831, 3244 to 3262, 3315 to 3333, 3423 to 3442, 4266 to 4284, 4328 to 4346, 4365 to 4400, 4714 to 4751, 4776 to 4795, 4949 to 4968 in the nucleotide sequence set forth in SEQ ID NO: 1; or an antisense oligonucleotide consisting of a nucleotide sequence in which one to three bases, one to two bases, or one base are substituted, deleted, or inserted with regard to said antisense oligonucleotide.

Examples of the antisense oligonucleotide consisting of a 11- to 19-base-long nucleotide sequence complementary to the nucleotide sequence at positions 348 to 366 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 3, or an antisense oligonucleotide consisting of a nucleotide sequence in which one to three bases are substituted, deleted, or inserted with regard to said antisense oligonucleotide. More specific examples include an antisense oligonucleotide depicted as hSYT13-350-AmNA (15) in Examples.

Examples of the antisense oligonucleotide consisting of a 11- to 19-base-long nucleotide sequence complementary to the nucleotide sequence at positions 599 to 627 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in any one of SEQ ID NOs: 4 and 50 to 59, or an antisense oligonucleotide consisting of a nucleotide sequence in which one to three bases are substituted, deleted, or inserted with regard to said antisense oligonucleotide. More specific examples include an antisense oligonucleotide depicted as hSYT13-605-AmNA(15), hSYT13-607-AmNA(13), hSYT13-609-AmNA(13), hSYT13-603-AmNA(15), hSYT13-607-AmNA(15), hSYT13-609-AmNA(15), hSYT13-601-AmNA(17), hSYT13-603-AmNA(17), hSYT13-605-AmNA(17), hSYT13-607-AmNA(17), or hSYT13-609-AmNA(17) in Examples.

Examples of the antisense oligonucleotide consisting of a 11- to 19-base-long nucleotide sequence complementary to the nucleotide sequence at positions 997 to 1016 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 5 or 6, or an antisense oligonucleotide consisting of a nucleotide sequence in which one to three bases are substituted, deleted, or inserted with regard to said antisense oligonucleotide. More specific examples include an antisense oligonucleotide depicted as hSYT13-999-AmNA (15) or hSYT13-1000-AmNA (15) in Examples.

Examples of the antisense oligonucleotide consisting of a 11- to 19-base-long nucleotide sequence complementary to the nucleotide sequence at positions 1069 to 1088 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 7 or 8, or an antisense oligonucleotide consisting of a nucleotide sequence in which one to three bases are substituted, deleted, or inserted with regard to said antisense oligonucleotide. More specific examples include an antisense oligonucleotide depicted as hSYT13-1071-AmNA (15) or hSYT13-1072-AmNA (15) in Examples.

Examples of the antisense oligonucleotide consisting of a 11- to 19-base-long nucleotide sequence complementary to the nucleotide sequence at positions 1419 to 1437 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 9, or an antisense oligonucleotide consisting of a nucleotide sequence in which one to three bases are substituted, deleted, or inserted with regard to said antisense oligonucleotide. More specific examples include an antisense oligonucleotide depicted as hSYT13-1421-AmNA (15) in Examples.

Examples of the antisense oligonucleotide consisting of a 11- to 19-base-long nucleotide sequence complementary to the nucleotide sequence at positions 1612 to 1641 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in any one of SEQ ID NOS: 10 to 16, or an antisense oligonucleotide consisting of a nucleotide sequence in which one to three bases are substituted, deleted, or inserted with regard to said antisense oligonucleotide. More specific examples include an antisense oligonucleotide depicted as hSYT13-1614-AmNA (15), hSYT13-1617-AmNA (15), hSYT13-1618-AmNA (15), hSYT13-1619-AmNA (15), hSYT13-1622-AmNA (15), hSYT13-1623-AmNA (15), or hSYT13-1625-AmNA (15) in Examples.

Examples of the antisense oligonucleotide consisting of a 11- to 19-base-long nucleotide sequence complementary to the nucleotide sequence at positions 1775 to 1793 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 17, or an antisense oligonucleotide consisting of a nucleotide sequence in which one to three bases are substituted, deleted, or inserted with regard to said antisense oligonucleotide. More specific examples include an antisense oligonucleotide depicted as hSYT13-1777-AmNA (15) in Examples.

Examples of the antisense oligonucleotide consisting of a 11- to 19-base-long nucleotide sequence complementary to the nucleotide sequence at positions 2629 to 2647 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 18, or an antisense oligonucleotide consisting of a nucleotide sequence in which one to three bases are substituted, deleted, or inserted with regard to said antisense oligonucleotide. More specific examples include an antisense oligonucleotide depicted as hSYT13-2631-AmNA (15) in Examples.

Examples of the antisense oligonucleotide consisting of a 11- to 19-base-long nucleotide sequence complementary to the nucleotide sequence at positions 2810 to 2831 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in any one of SEQ ID NOs: 19 to 22, or an antisense oligonucleotide consisting of a nucleotide sequence in which one to three bases are substituted, deleted, or inserted with regard to said antisense oligonucleotide. More specific examples include an antisense oligonucleotide depicted as hSYT13-2812-AmNA (15), hSYT13-2813-AmNA (15), hSYT13-2814-AmNA (15), or hSYT13-2815-AmNA (15) in Examples.

Examples of the antisense oligonucleotide consisting of a 11- to 19-base-long nucleotide sequence complementary to the nucleotide sequence at positions 3244 to 3262 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 23, or an antisense oligonucleotide consisting of a nucleotide sequence in which one to three bases are substituted, deleted, or inserted with regard to said antisense oligonucleotide. More specific examples include an antisense oligonucleotide depicted as hSYT13-3246-AmNA (15) in Examples.

Examples of the antisense oligonucleotide consisting of a 11- to 19-base-long nucleotide sequence complementary to the nucleotide sequence at positions 3315 to 3333 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 24, or an antisense oligonucleotide consisting of a nucleotide sequence in which one to three bases are substituted, deleted, or inserted with regard to said antisense oligonucleotide. More specific examples include an antisense oligonucleotide depicted as hSYT13-3317-AmNA (15) in Examples.

Examples of the antisense oligonucleotide consisting of a 11- to 19-base-long nucleotide sequence complementary to the nucleotide sequence at positions 3423 to 3442 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 25 or 26, or an antisense oligonucleotide consisting of a nucleotide sequence in which one to three bases are substituted, deleted, or inserted with regard to said antisense oligonucleotide. More specific examples include an antisense oligonucleotide depicted as hSYT13-3425-AmNA (15) or hSYT13-3426-AmNA (15) in Examples.

Examples of the antisense oligonucleotide consisting of a 11- to 19-base-long nucleotide sequence complementary to the nucleotide sequence at positions 4266 to 4284 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 27, or an antisense oligonucleotide consisting of a nucleotide sequence in which one to three bases are substituted, deleted, or inserted with regard to said antisense oligonucleotide. More specific examples include an antisense oligonucleotide depicted as hSYT13-4268-AmNA (15) in Examples.

Examples of the antisense oligonucleotide consisting of a 11- to 19-base-long nucleotide sequence complementary to the nucleotide sequence at positions 4328 to 4346 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 28, or an antisense oligonucleotide consisting of a nucleotide sequence in which one to three bases are substituted, deleted, or inserted with regard to said antisense oligonucleotide. More specific examples include an antisense oligonucleotide depicted as hSYT13-4330-AmNA (15) in Examples.

Examples of the antisense oligonucleotide consisting of a 11- to 19-base-long nucleotide sequence complementary to the nucleotide sequence at positions 4365 to 4400 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in any one of SEQ ID NOs: 29 to 36 and 60 to 69, or an antisense oligonucleotide consisting of a nucleotide sequence in which one to three bases are substituted, deleted, or inserted with regard to said antisense oligonucleotide. More specific examples include an antisense oligonucleotide depicted as hSYT13-4367-AmNA(15), hSYT13-4368-AmNA(15), hSYT13-4371-AmNA(15), hSYT13-4373-AmNA(15), hSYT13-4374-AmNA(15), hSYT13-4377-AmNA(15), hSYT13-4378-AmNA(15), hSYT13-4381-AmNA(15), hSYT13-4374-AmNA(13), hSYT13-4376-AmNA(15), hSYT13-4380-AmNA(15), hSYT13-4382-AmNA(15), hSYT13-4374-AmNA(17), hSYT13-4376-AmNA(17), hSYT13-4378-AmNA(17), hSYT13-4380-AmNA(17), hSYT13-4382-AmNA(17), or hSYT13-4374-AmNA(19) in Examples.

Examples of the antisense oligonucleotide consisting of a 11- to 19-base-long nucleotide sequence complementary to the nucleotide sequence at positions 4714 to 4751 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in any one of SEQ ID NOs: 37 to 39 and 70 to 79, or an antisense oligonucleotide consisting of a nucleotide sequence in which one to three bases are substituted, deleted, or inserted with regard to said antisense oligonucleotide. More specific examples include an antisense oligonucleotide depicted as hSYT13-4716-AmNA(15), hSYT13-4717-AmNA(15), hSYT13-4729-AmNA(15), hSYT13-4725-AmNA(13), hSYT13-4727-AmNA(13), hSYT13-4725-AmNA(15), hSYT13-4727-AmNA(15), hSYT13-4731-AmNA(15), hSYT13-4725-AmNA(17), hSYT13-4727-AmNA(17), hSYT13-4729-AmNA(17), hSYT13-4731-AmNA(17), or hSYT13-4733-AmNA(17) in Examples. Further, an antisense oligonucleotide depicted as 4733-A, 4733-B, 4733-C, 4733-D, 4733-E, 4733-F, 4733-G, 4733-H, 4733-1, 4733-J, 4733-K, 4733-L, 4733-M, or 4733-N in Examples is also included.

Examples of the antisense oligonucleotide consisting of a 11- to 19-base-long nucleotide sequence complementary to the nucleotide sequence at positions 4776 to 4795 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 40 or 41, or an antisense oligonucleotide consisting of a nucleotide sequence in which one to three bases are substituted, deleted, or inserted with regard to said antisense oligonucleotide. More specific examples include an antisense oligonucleotide depicted as hSYT13-4778-AmNA (15) or hSYT13-4779-AmNA (15) in Examples.

Examples of the antisense oligonucleotide consisting of a 11- to 19-base-long nucleotide sequence complementary to the nucleotide sequence at positions 4949 to 4968 in the nucleotide sequence set forth in SEQ ID NO: 1, or the antisense oligonucleotide consisting of a nucleotide sequence in which one to three bases are substituted, deleted, or inserted with regard to said antisense oligonucleotide include: an antisense oligonucleotide consisting of a nucleotide sequence set forth in SEQ ID NO: 42 or 43, or an antisense oligonucleotide consisting of a nucleotide sequence in which one to three bases are substituted, deleted, or inserted with regard to said antisense oligonucleotide. More specific examples include an antisense oligonucleotide depicted as hSYT13-4951-AmNA (15) or hSYT13-4952-AmNA (15) in Examples.

In one preferred embodiment, the nucleotide sequence of the antisense oligonucleotide may be a nucleotide sequence selected from the group consisting of SEQ ID NOs: 3 to 43, or a nucleotide sequence in which one to three bases are substituted, deleted, or inserted with regard to said nucleotide sequence. In this embodiment, more specifically, it is preferable that the antisense oligonucleotide be one shown in Table 1 below.

In another preferred embodiment, the nucleotide sequence of the antisense oligonucleotide may be a nucleotide sequence selected from the group consisting of SEQ ID NOs: 50 to 59, or a nucleotide sequence in which one to three bases are substituted, deleted, or inserted with regard to said nucleotide sequence. In this embodiment, more specifically, it is preferable that the antisense oligonucleotide be one shown in Table 2 below.

In another preferred embodiment, the nucleotide sequence of the antisense oligonucleotide may be a nucleotide sequence selected from the group consisting of SEQ ID NOs: 60 to 69, or a nucleotide sequence in which one to three bases are substituted, deleted, or inserted with regard to said nucleotide sequence. In this embodiment, more specifically, it is preferable that the antisense oligonucleotide be one shown in Table 3 below.

In another preferred embodiment, the nucleotide sequence of the antisense oligonucleotide may be a nucleotide sequence selected from the group consisting of SEQ ID NOs: 70 to 79, or a nucleotide sequence in which one to three bases are substituted, deleted, or inserted with regard to said nucleotide sequence. In this embodiment, more specifically, it is preferable that the antisense oligonucleotide be one shown in Table 4 below.

In another preferred embodiment, the nucleotide sequence of the antisense oligonucleotide may be a nucleotide sequence selected from the group consisting of SEQ ID NOs: 4, 20, 29, 35, 39, 62, and 79, or a nucleotide sequence in which one to three bases are substituted, deleted, or inserted with regard to said nucleotide sequence.

In another preferred embodiment, the nucleotide sequence of the antisense oligonucleotide may be a nucleotide sequence selected from the group consisting of SEQ ID NOs: 80 to 93, or a nucleotide sequence in which one to three bases are substituted, deleted, or inserted with regard to said nucleotide sequence. In this embodiment, more specifically, it is preferable that the antisense oligonucleotide be one shown in Table 5 below.

Sequence homology can be analyzed using algorithms known in this technical field, for example, by BLAST analysis (see, for example, Altschul, S.F., et al., Basic local alignment search tool. 1990, J. Mol. Biol. 215: 403-410).

The antisense oligonucleotide of the present invention may be in a range of from 11 to 20 bases in length and may be, for example, 12 to 19 bases in length, 14 to 18 bases in length, or 15 to 17 bases in length.

In the present invention, the antisense oligonucleotide may comprise a native (unmodified) nucleotide (deoxyribonucleotide, ribonucleotide, or both) and/or a non-native (modified) nucleotide.

In general, a "nucleoside" is a combination of a sugar and a nucleobase. A "nucleotide" further comprises a phosphate group covalently bonded to the sugar moiety of a nucleoside. Phosphate groups typically form internucleoside linkages in oligonucleotides. An oligonucleotide is formed by covalent bonding of nucleosides adj acent to each other, and a linear polymer oligonucleotide is thus formed.

The term "modified nucleoside" as used herein refers to a nucleoside independently having a modified sugar and/or a modified nucleobase. The term "modified nucleotide" as used herein refers to a nucleotide independently having a modified internucleoside linkage, a modified sugar and/or a modified nucleobase. The oligonucleotide comprising a modified nucleotide is preferable to an unmodified form because of desirable properties such as an enhanced affinity for a target nucleic acid and increased resistance to nucleases.

The term "modified internucleoside linkage" as used herein refers to an internucleoside linkage that is a replacement of or has some change from the native internucleoside linkage (i.e., a phosphodiester bond). Examples of the modified internucleoside linkage include, but are not limited to, a phosphorothioate bond, a phosphorodithioate bond, a phosphorodiamidate bond, and a phosphoramidate bond. The phosphorothioate bond refers to an internucleoside linkage in which the non-bridging oxygen atom in the phosphodiester bond is replaced by a sulfur atom. The modified internucleoside linkage is preferably a linkage with higher resistance to nucleases than the native internucleoside linkage.

The term "modified nucleobase" as used herein refers to any nucleobase other than adenine, cytosine, guanine, thymine, or uracil. The term "unmodified nucleobase" or "native nucleobase" refers to the purine bases adenine (A) and guanine (G), and the pyrimidine bases thymine (T), cytosine (C), and uracil (U). Examples of the modified nucleobase include, but are not limited to, 5-methylcytosine, 5-fluorocytosine, 5-bromocytosine, or 5-iodocytosine; 5-fluorouracil, 5-bromouracil, 5-iodouracil, or 5-hydroxyuracil; 2-thiothymine; N6-methyladenine or 8-bromoadenine; and N2-methylguanine or 8-bromoguanine.

The term "modified sugar" as used herein refers to a sugar that has a replacement of or has some change from a native sugar moiety (i.e., the sugar moiety found in DNA(2'-H) or RNA(2'-OH)). The modified sugar can provide the oligonucleotide with an enhanced affinity for a target nucleic acid and increased resistance to nucleases. Examples of the modified sugar include bicyclic sugar, 5'-vinyl, 5'-methyl, 4'-S, 2'-F, 2'-OCH₃ (2'-methoxy or 2'-O-methyl group), and 2'-O(CH₂)₂OCH₃ substituents.

The term "bicyclic sugar" as used herein refers to a sugar with two rings. A nucleic acid containing a bicyclic sugar moiety is commonly referred to as a bridged nucleic acid (BNA). The bicyclic sugar may be a sugar in which the carbon atom at position 2' and the carbon atom at position 4' are bridged by two or more atoms. Examples of the bicyclic sugar include, but are not limited to, sugars having a methyleneoxy (4'-CH₂-O-2') bridge (LNA^{™}, also known as 2',4'-BNA), sugars having an ethyleneoxy (4'-(CH₂)₂-O-2') bridge (also known as ENA), sugars having a 4'-CH(CH₃)-O-2' bridge (cEt, constrained ethyl), sugars having a 4'-CH(CH₂OCH₃)-O-2' bridge (cMOE, constrained MOE), and sugars having an amide bridge (AmNA, Amido-bridged nucleic acid).

One example of the sugars having an amide bridge is a sugar having a 4'-C(O)-N(CH₃)-2' bridge. For structures of and preparation methods for the sugars having an amide bridge, see, for example, Yahara, A., et al., Amido-bridged nucleic acids (AmNAs): synthesis, duplex stability, nuclease resistance, and in vitro antisense potency, ChemBioChem, 2012, 13(7): 2513-2516, Yamamoto, T., et al., Amido-bridged nucleic acids with small hydrophobic residues enhance hepatic tropism of antisense oligonucleotides in vivo, Org. Biomol. Chem., 2015, 13:3757-3765, and International Publication No. WO 2011/052436. For sugars having a 4'-CH(CH₃)-O-2' bridge (cEt) and sugars having a 4'-CH(CH₂OCH₃)-O-2' bridge (cMOE), see Punit, P.S., et al., Short antisense oligonucleotides with novel 2'-4' conformationally restricted nucleoside analogues show improved potency without increased toxicity in animals, J. Med. Chem., 2009, 52(1): 10-13.

The antisense oligonucleotide may also comprise a nucleotide mimetic such as peptide nucleic acids and morpholino nucleic acids.

In general, different nucleotides in the same chain can independently undergo different modifications. Also, for example, to enhance the nuclease resistance, the same nucleotide can have a modified internucleoside linkage (e.g., a phosphorothioate bond) and can further have a modified sugar (e.g., a bicyclic sugar). The same nucleotide can also have a modified nucleobase (e.g., 5-methylcytosine) and can further have a modified sugar (e.g., a bicyclic sugar).

In one embodiment, the antisense oligonucleotide may contain at least one modified nucleotide. The modified nucleotide may include a modified internucleoside linkage, a modified sugar moiety, and/or a modified nucleobase.

In one embodiment, at least one of the internucleoside linkages in the antisense oligonucleotide may be a modified internucleoside linkage. At least 70%, at least 80%, at least 90%, or 100% of the internucleoside linkage in the antisense oligonucleotide may be modified internucleoside linkages. The modified internucleoside linkage may be a phosphorothioate bond.

In one embodiment, at least one of the sugar moieties of the antisense oligonucleotide may be a bicyclic sugar. The bicyclic sugar may have a methyleneoxy (4'-CH₂-O-2') bridge or an amide bridge (e.g., 4'-C(O)-N(CH₃)-2' bridge). In the present invention, the antisense oligonucleotide having an amide bridge can be suitably used.

In one embodiment, at least one of the nucleobases of the antisense oligonucleotide may be a modified nucleobase. The modified nucleobase may be 5-methylcytosine.

In a specific embodiment, the antisense oligonucleotide may be a gapmer. The term "gapmer" as used herein refers to an oligonucleotide consisting of a central region containing at least four contiguous deoxyribonucleosides (DNA gap region) and regions containing non-native nucleosides located at the 5' and 3' ends of the central region (a 5' wing region and a 3' wing region). The DNA gap region may be 4 to 16 bases in length, 5 to 14 bases in length, 6 to 12 bases in length, or 8 to 10 bases in length. The 5' wing region and the 3' wing region may be independently 1 to 6 bases in length, 1 to 5 bases in length, or 2 to 4 bases in length. The 5' wing region and the 3' wing region contain at least one non-native nucleoside and may contain a native nucleoside. The 5' wing region and the 3' wing region may each contain one or more types of non-native nucleosides. All nucleosides in the 5' wing region and the 3' wing region may be non-native nucleosides. Alternatively, nucleosides at either or both of the 5' and 3' ends (especially at the 3' end) of the gapmer may be native nucleosides (especially deoxyribonucleosides). A non-native nucleoside contained in the 5' wing region and the 3' wing region may be a nucleoside having a bicyclic sugar. The bicyclic sugar may be a sugar having a methyleneoxy (4'-CH₂-O-2') bridge or a sugar having an amide bridge (e.g., 4'-C(O)-N(CH₃)-2' bridge). A non-native nucleoside contained in the 5' wing region and the 3' wing region may contain a modified nucleobase (e.g., 5-methylcytosine).

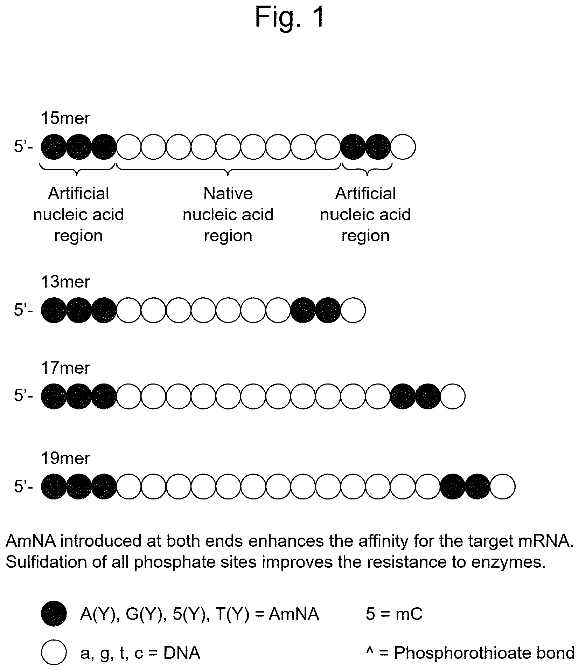

In a suitable embodiment, the antisense oligonucleotide is a gapmer that is used in Examples below. Figure 1 shows an example structure of the antisense oligonucleotide that can be suitably used in the present invention; however, the structure of a suitable gapmer is not limited thereto, and modification patterns for the sugar, nucleobase, and/or internucleoside linkage may be different.

The antisense oligonucleotide of the present invention can be produced by methods known in the art. For example, the antisense oligonucleotide can be synthesized using a commercially available automated nucleic acid synthesizer, and then purified using a reversephase column. Alternatively, the antisense oligonucleotide can be ordered and obtained from a manufacturer (e.g., Gene Design, Inc.) by designating a nucleobase sequence and a modification site and type.

The antisense oligonucleotide of the present invention inhibits the expression of human SYT13 gene, and thus can be used as a medicine that inhibits the peritoneal dissemination in gastric cancer.

The antisense oligonucleotide of the present invention can inhibit the proliferation, migration, and/or invasion of cancer cells expressing SYT13 *in vitro* or *in vivo.* Delivery of the antisense oligonucleotide into cells can be performed using any method commonly used in this field, such as lipofection, electroporation, microinjection, particle gun, and transduction using viruses or plasmids as vectors. Alternatively, the antisense oligonucleotide can be transfected directly into cells. For example, the antisense oligonucleotide can be suitably delivered into cells *in vitro* and *in vivo* using the CEM method (Nucleic Acids Research, 2015, Vol. 43, No. 19, e128; doi: 10.1093/nar/gkv626).

The antisense oligonucleotide of the present invention inhibits the expression of human SYT13 gene and thus can be used as a medicine that inhibits peritoneal dissemination of gastric cancer. The efficacy of the present invention is demonstrated in the Examples below.

### (Conjugate)

The present invention also provides a conjugate comprising the above-described antisense oligonucleotide, and a further functional moiety directly or indirectly linked each other.

The further functional moiety contemplated in the present invention may be a small molecule such as a peptide, sugar, or lipid, and may be, but is not limited to, a ligand for target molecule or an agent with antitumor activity. More specifically, the functional molecule may be, for example, a binding molecule such as an antibody or antigen-binding fragment thereof to a protein that can be highly expressed at a tumor site, GalNAc that can bind to glycoprotein receptors, or a lipid such as cholesterol or a long-chain fatty acid that can enhance cell membrane permeability. In addition, the functional molecule may be, for example, an agent with another antitumor activity.

The antisense oligonucleotide and the further functional moiety may be linked directly or via a linker commonly used in this field. The linkage is preferably, but is not limited to, a covalent bond. Administering the antisense oligonucleotide of the present invention as the conjugate can facilitate the delivery to its target site and/or improve the efficacy of the antisense oligonucleotide of the present invention.

### (Pharmaceutical composition)

The present invention provides a pharmaceutical composition comprising the antisense oligonucleotide or conjugate of the present invention. The pharmaceutical composition of the present invention can be used, for example, to prevent or treat peritoneal dissemination after gastric cancer resection.

The pharmaceutical composition may further comprise any formulation aids normally used in the field of pharmaceutical formulation. As the formulation aids herein, various carriers or additives such as pharmaceutically acceptable carriers (solid or liquid carriers), excipients, stabilizers, disintegrators, surfactants, binders, lubricants, emulsifiers, suspension agents, antioxidants, odor-masking agents, fillers, solubilizers, coating agents, colorants, tastemasking agents, preservatives, and buffers can be used. Specific examples of the formulation aids include water, physiological saline, other aqueous solvents, pharmaceutically acceptable organic solvents, mannitol, lactose, starch, microcrystalline cellulose, glucose, calcium, polyvinyl alcohol, collagen, polyvinylpyrrolidone, carboxyvinyl polymer, sodium alginate, water-soluble dextran, water-soluble dextrin, sodium carboxymethyl starch, pectin, gum arabic, xanthan gum, casein, gelatin, agar, propylene glycol, polyethylene glycol, vaseline, paraffin, glycerin, stearyl alcohol, stearic acid, and sorbitol. The formulation aid may be selected as appropriate or in combination depending on the dosage form of the pharmaceutical formulation.

The pharmaceutical composition can be administered to a subject orally or parenterally. Examples of parenteral administration include, but are not limited to, intraperitoneal administration. In order to efficiently attain the therapeutic effect, it is preferable that the pharmaceutical composition be administered directly and topically to a damaged site. The pharmaceutical composition can also be continuously administered to the damaged site using a continuous infusion pump. The pharmaceutical composition may be formulated into an injection, a drip, or the like. Those skilled in the art can produce these pharmaceutical formulations in a conventional manner.

The pharmaceutical composition may be administered in a therapeutically effective amount. A specific dosage of the pharmaceutical composition is determined by the physician, for example, based on the disease severity, general health condition, age, sex, body weight, tolerability to the treatment, etc., according to individual subjects. For example, the pharmaceutical composition may be administered such that the dose of the antisense oligonucleotide is 0.000001 mg/kg body weight/day to 1000 mg/kg body weight/day, or 0.001 mg/kg body weight/day to 1 mg/kg body weight/day, or 0.005 mg/kg body weight/day to 0.5 mg/kg body weight/day, or 0.01 mg/kg body weight/day to 0.1 mg/kg body weight/day. The pharmaceutical composition can be administered as a single dose or multiple doses and may be administered to a subject several times or several tens of times, for example, at regular time intervals such as 1 day, 2 days, 3 days, 4 days, 5 days, 6 days, 1 week, 2 weeks, 3 weeks, or 1 month. Alternatively, the pharmaceutical composition may be administered continuously using a continuous infusion pump as described above. The dosage (rate), duration, etc. for the continuous administration can be set appropriately by those skilled in the art.

The subject to which the pharmaceutical composition is administered is a mammal such as primates (e.g., cynomolgus monkeys, chimpanzees, and humans) and non-primates (e.g., cattle, pigs, sheep, horses, cats, dogs, guinea pigs, rats, and mice), and is more preferably a human. The subject may be, for example, an animal model for gastric cancer transplanted with human gastric cancer cells.

The present invention also provides a method for preventing or treating peritoneal dissemination after gastric cancer resection, the method comprising administering the antisense oligonucleotide, conjugate, or pharmaceutical composition of the present invention to a subject in need thereof.

The present invention also provides use of the antisense oligonucleotide or conjugate of the present invention in the manufacture of a medicine for preventing or treating peritoneal dissemination after gastric cancer resection.

### Examples

Hereinafter, the present invention will be described in more detail using Examples. However, the technical scope of the present invention is not limited to these Examples.

### [Example 1 Selection of candidate antisense oligonucleotide sequences]

As a target region of an antisense oligonucleotide, a region with a sequence commonly included in the mRNA sequences of two variants of SYT13, NM_020826.2 (SEQ ID NO: 1) and NM_001247987.1 (SEQ ID NO: 2) was extracted. At this point, thousands of candidate target sequences were obtained.

Next, after their homology with mouse SYT13 mRNA was examined by BLAST, the conformations of antisense oligonucleotides to be obtained were predicted to exclude sequences that are expected to have a high risk of toxicity manifestation, whereby several hundreds of candidate sequences were selected.

After sequences for antisense strands were obtained from the selected sequences and the candidate sequences were further narrowed down by selection based on their physical properties as antisense oligonucleotides, sequences with a high risk of off-target were excluded to select 41 candidate sequences. Based on these sequences, their respective antisense oligonucleotide molecules were designed and synthesized.

The structure of the antisense oligonucleotide used in this Example is shown in Figure 1. For example, when designing a 15-mer antisense oligonucleotide, artificial nucleic acid regions with three bases and two bases were provided respectively on the 5' side and the 3' side, and a native nucleic acid region was provided therebetween. In this Example, amide-bridged nucleic acid (AmNA) with the following sugar structure was used as the artificial nucleic acid (A. Yahara et al., ChemBioChem, 2012, 13, 2513-2516; T. Yamamoto et al., Org. Biomol. Chem., 2015, 13, 3757-3765).

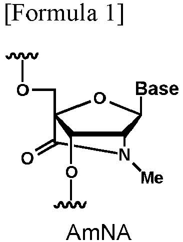

In the above structure, "Base" means a base, such as adenine, cytosine, guanine, or thymine, but can include a modified base such as 5-methylcytosine in the artificial nucleic acid region.

In Figure 1, a black circle in the artificial nucleic acid region indicates that AmNA is used as the sugar instead of deoxyribose found in native nucleic acids, and a white circle in the native nucleic acid region indicates that deoxyribose is used. It was confirmed that the introduction of AmNA at both ends of the antisense oligonucleotide molecule can improve the affinity for the target mRNA. In this Example, 5-methylcytosine was used as the base for the artificial nucleic acid region instead of cytosine found in native nucleic acids.

The antisense oligonucleotide used in this Example was prepared to have a phosphorothioate bond instead of a phosphodiester bond found in native nucleic acids to improve its resistance to enzymes.

Table 1 below shows sequence information for antisense oligonucleotides designed based on the candidate sequences selected above and for control antisense oligonucleotides (NEG1 and NEG2) designed not to bind to any of the known genes. For example, "hSYT13-350-AmNA (15)" in Table 1 has a sequence complementary to the consecutive 15 bases from position 350 in SEQ ED NO: 1, which is a nucleotide sequence of human SYT13 mRNA. In Table 1, underlines indicate mismatched bases to mouse SYT13 mRNA.

**[Table 1]**

| Sequence information for antisense nucleic acids designed | | |
|---|---|---|
| Sequence Name | Antisense (5'→3') | SEQ ID NO: |
| hSYT13-350-AmNA(15) | G(Y)^G(Y)^A(Y)^a^c^t^t^g^a^g^g^a^G(Y)^G(Y)^g | 3 |
| hSYT13-605-AmNA(15) | A(Y)^G(Y)^T(Y)^a^g^t^g^g^a^g^t^t^T(Y)^G(Y)^g | 4 |
| hSYT13-999-AmNA(15) | 5(Y)^T(Y)^G(Y)^a^t^g^g^a^t^a^g^t^A(Y)^G(Y)^g | 5 |
| hSYT13-1000-AmNA(15) | G(Y)^5(Y)^T(Y)^g^a^t^g^g^a^t^a^g^T(Y)^A(Y)^g | 6 |
| hSYT13-1071-AmNA(15) | T(Y)^T(Y)^G(Y)^g^a^c^t^g^g^t^t^a^G(Y)^A(Y)^g | 7 |
| hSYT13-1072-AmNA(15) | 5(Y)^T(Y)^T(Y)^g^g^a^c^t^g^g^t^t^A(Y)^G(Y)^a | 8 |
| hSYT13-1421-AmNA(15) | 5 (Y) ^5(Y) ^A(Y) ^a^g^a^a^g^g^g^a^g^G(Y) ^5(Y) ^a | 9 |
| hSYT13-1614-AmNA(15) | A(Y)^5(Y)^A(Y)^g^a^t^g^a^g^c^a^a^A(Y)^A(Y)^t | 10 |
| hSYT13-1617-AmNA(15) | A(Y)^A(Y)^5(Y)^a^c^a^g^a^t^g^a^g^5(Y)^A(Y)^a | 11 |
| hSYT13-1618-AmNA(15) | T(Y)^A(Y)^A(Y)^c^a^c^a^g^a^t^g^a^G(Y)^5(Y)^a | 12 |
| hSYT13-1619-AmNA(15) | A(Y)^T(Y)^A(Y)^a^c^a^c^a^g^a^t^g^A(Y)^G(Y)^c | 13 |
| hSYT13-1622-AmNA(15) | T(Y)^5(Y)^A(Y)^a^t^a^a^c^a^c^a^g^A(Y)^T(Y)^g | 14 |
| hSYT13-1623-AmNA(15) | T(Y)^T(Y)^5(Y)^a^a^t^a^a^c^a^c^a^G(Y)^A(Y)^t | 15 |
| hSYT13-1625-AmNA(15) | 5(Y)^5(Y)^T(Y)^t^c^a^a^t^a^a^c^a^5(Y)^A(Y)^g | 16 |
| hSYT13-1777-AmNA(15) | G(Y)^A(Y)AT(Y)^a^c^t^t^t^c^a^c^t^T(Y)^5(Y)^c | 17 |
| hSYT13-2631-AmNA(15) | A(Y)^A(Y)^G(Y)^g^c^t^c^a^t^a^a^t^T(Y)^T(Y)^a | 18 |
| hSYT13-2812-AmNA(15) | T(Y)^T(Y)^T(Y)^t^t^a^g^c^c^a^g^a^G(Y)^A(Y)^g | 19 |
| hSYT13-2813-AmNA(15) | G(Y)^T(Y)^T(Y)^t^t^t^a^g^c^c^a^g^A(Y)^G(Y)^a | 20 |
| hSYT13-2814-AmNA(15) | T(Y)AG(Y)AT(Y)^t^t^t^t^a^g^c^c^a^G(Y)^A(Y)^g | 21 |
| hSYT13-2815-AmNA(15) | T(Y)^T(Y)^G(Y)^t^t^t^t^t^a^g^c^c^A(Y)^G(Y)^a | 22 |
| hSYT13-3246-AmNA(15) | 5(Y)^5(Y)^A(Y)^a^a^g^g^c^a^g^a^a^T(Y)^G(Y)^c | 23 |
| hSYT13-3317-AmNA(15) | A(Y)^A(Y)^T(Y)^t^c^t^g^t^c^t^t^a^G(Y)^G(Y)^g | 24 |
| hSYT13-3425-AmNA(15) | G(Y)^A(Y)^A(Y)^a^a^a^a^t^a^a^t^g^A(Y)^5(Y)^c | 25 |
| hSYT13-3426-AmNA(15) | A(Y)^G(Y)^A(Y)^a^a^a^a^a^t^a^a^t^G(Y)^A(Y)^c | 26 |
| hSYT13-4268-AmNA(15) | T(Y)^T(Y)^T(Y)^a^c^c^a^t^t^g^a^g^A(Y)^A(Y)^c | 27 |
| hSYT13-4330-AmNA(15) | T(Y)^A(Y)^G(Y)^g^c^a^a^a^t^a^g^t^A(Y) ^A(Y) ^t | 28 |
| hSYT13-4367-AmNA(15) | G(Y)^T(Y)^T(Y)^g^a^t^t^a^c^a^t^t^T(Y)^A(Y)^c | 29 |
| hSYT13-4368-AmNA(15) | T(Y) ^G(Y) ^T(Y) ^t^g^a^t^t^a^c^a^t^T(Y) ^T(Y) ^a | 30 |
| hSYT13-4371-AmNA(15) | A(Y)^T(Y)^5(Y)^t^g^t^t^g^a^t^t^a^5(Y)^A(Y)^t | 31 |
| hSYT13-4373-AmNA(15) | T(Y)^5(Y)^A(Y)^t^c^t^g^t^t^g^a^t^T(Y)^A(Y)^c | 32 |
| hSYT13-4374-AmNA(15) | T(Y)^T(Y)^5(Y)^a^t^c^t^g^t^t^g^a^T(Y)^T(Y)^a | 33 |
| hSYT13-4377-AmNA(15) | 5(Y)^T(Y)^5(Y)^t^t^c^a^t^c^t^g^t^T(Y)^G(Y)^a | 34 |
| hSYT13-4378-AmNA(15) | T(Y)^5(Y)^T(Y)^c^t^t^c^a^t^c^t^g^T(Y)^T(Y)^g | 35 |
| hSYT13-4381-AmNA(15) | T(Y)^A(Y)^T(Y)^t^c^t^c^t^t^c^a^t^5(Y)^T(Y)^g | 36 |
| hSYT13-4716-AmNA(15) | 5(Y)^5(Y) ^5(Y) ^g^a^t^t^t^t^c^t^a^T(Y) ^5(Y) ^c | 37 |
| hSYT13-4717-AmNA(15) | T(Y)^5(Y)^5(Y)^c^g^a^t^t^t^t^c^t^A(Y)^T(Y)^c | 38 |
| hSYT13-4729-AmNA(15) | A(Y)^T(Y)^G(Y)^a^g^g^g^g^a^g^a^c^T(Y)^5(Y)^c | 39 |
| hSYT13-4778-AmNA(15) | G(Y)^5(Y)^T(Y)^c^a^a^c^a^a^a^t^a^G(Y)^A(Y)^t | 40 |
| hSYT13-4779-AmNA(15) | T(Y)^G(Y)^5(Y)^t^c^a^a^c^a^a^a^t^A(Y)^G(Y)^a | 41 |
| hSYT13-4951-AmNA(15) | 5(Y)^A(Y)^5(Y)^a^t^t^t^t^a^c^c^c^A(Y)^G(Y)^g | 42 |
| hSYT13-4952-AmNA(15) | 5(Y)^5(Y)^A(Y)^c^a^t^t^t^t^a^c^c^5(Y)^A(Y)^g | 43 |
| NEG1 | 5(Y)^A(Y)^5(Y)^a^g^t^a^t^c^t^a^t^G(Y)^T(Y)^a | 44 |
| NEG2 | G(Y)^A(Y)^A(Y)^t^c^t^c^a^c^a^g^t^A(Y) ^T(Y)^c | 45 |

| | | |
|---|---|---|
| A(Y), G(Y), 5(Y), T(Y) = AmNA a, g, t, c = DNA ^ = Phosphorothioate bond 5 = mC | | |

The antisense oligonucleotides were synthesized according to a method commonly used in this field.

### [Example 2 Screening based on the inhibitory effect on the expression of SYT13 mRNA]

Human signet ring cell carcinoma-derived gastric cancer cell line KATOIII (obtained from American Type Culture Collection, ATCC) cultured at 37°C under 5% CO₂ in a medium prepared by mixing RPMI1640 (Nacalai Tesque Inc.) and DMEM (Nacalai Tesque Inc., Low-Glucose) at 1:1 and adding thereto 10% fetal bovine serum (FBS, biowest) and 1% penicillin-streptomycin mixed solution (Nacalai Tesque Inc., Stabilized) was seeded at a concentration of 10,000 cells/100 µL per well in DMEM containing 10% FBS in a 96-well plate, and cultured at 37°C under 5% CO₂ for 24 hours.

Cell transfection was performed by the CEM method. Specifically, 900 mM calcium chloride was added to the DMEM containing 10% FBS to prepare a 100-fold dilution, and then the antisense oligonucleotide synthesized in Example 1 was added to obtain a final concentration of 6.25 nM, 25 nM, or 100 nM, and cells were further cultured at 37°C under 5% CO₂ for 24 hours.

RNA was extracted from the transfected cells and reverse-transcribed into cDNA using Cell Lysis & RT Kit (TOYOBO SuperPrep^{™}Cell Lysis & RT Kit for qPCR).

The obtained cDNA was subjected to real-time PCR (RT-PCR) using ABI PowerUp^{™} SYBR^{®} Green Master Mix and the following primers (200 nM each). PCR was performed using a thermal cycler (ABI StepOnePlus^{™} Real-Time PCR System) under the following conditions: 95°C for 30 seconds, followed by 45 cycles of 95°C for 3 seconds and 60°C for 30 seconds.

GAPDH forward primer: CGACAGTCAGCCGCATCTT (SEQ ID NO: 46)
GAPDH reverse primer: CCCAATACGACCAAATCCGTTG (SEQ ID NO: 47)
SYT13 forward primer: TGGTGGTGCTGATTAAAGCC (SEQ ID NO: 48)
SYT13 reverse primer: TGCTTCTTCTTCAGCTTCCG (SEQ ID NO: 49)

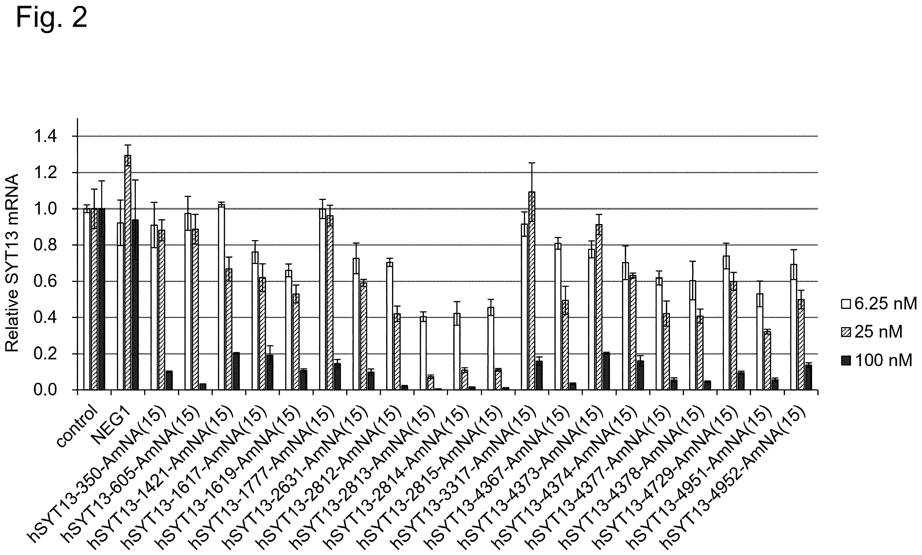

A relative expression level of SYT13 mRNA was calculated from the measured expression levels of SYT13 mRNA and GAPDH mRNA (a comparative control). Some of the results are shown in Figure 2.

As shown in Figure 2, it was demonstrated that the tested antisense oligonucleotides inhibit the expression of SYT13 mRNA in concentration-dependent manner.

### [Example 3 Demonstration of concentration-dependent inhibition of expression in various cells]

Human gastric cancer cell lines MKN1, MKN45, and OCUM-1 (obtained from the JCRB cell bank of the National Institutes of Biomedical Innovation, Health and Nutrition, Japan) were used to examine the efficacy of the antisense oligonucleotides synthesized in Example 1. For operational procedure for the cell culture and the like, those suitable for each cell line were used as appropriate but were substantially the same as in Example 2.

For MKN1 cells, MKN1 cells that were cultured in RPMI 1640 (Nacalai Tesque Inc.) supplemented with 10% FBS (biowest) and 1% penicillin-streptomycin mixed solution (Nacalai Tesque Inc., Stabilized) at 37°C under 5% CO₂ were seeded at a concentration of 8,000 cells/100 µL per well in DMEM containing 10% FBS in a 96-well plate, and cultured at 37°C under 5% CO₂ for 24 hours; subsequently, the antisense oligonucleotide synthesized in Example 1 was added to prepare a final concentration of 6.25 nM, 25 nM, or 100 nM, and cells were further cultured at 37°C under 5% CO₂ for 24 hours. For extraction of RNA from the transfected cells, RNA was extracted and reverse-transcribed into cDNA using Cell Lysis & RT Kit (TOYOBO SuperPrep^{™}Cell Lysis & RT Kit for qPCR).

For MKN45 cells, MKN45 cells that were cultured in RPMI 1640 (Nacalai Tesque Inc.) supplemented with 10% FBS (biowest) and 1% penicillin-streptomycin mixed solution (Nacalai Tesque Inc., Stabilized) at 37°C under 5% CO₂ were seeded at a concentration of 30,000 cells/500 µL per well in DMEM containing 10% FBS in a 24-well plate, and cultured at 37°C under 5% CO₂ for 24 hours; subsequently, the antisense oligonucleotide synthesized in Example 1 was added to prepare a final concentration of 50 nM or 200 nM, and cells were further cultured at 37°C under 5% CO₂ for 24 hours. Extraction of RNA from the transfected cells was performed using QIAGEN Rneasy^{®} Mini Kit (QIAGEN^{®}) and reverse-transcribed into cDNA using a high-capacity cDNA reverse transcription kit (ABI High-Capacity cDNA Reverse Transcription Kit).

For OCUM-1 cells, OCUM-1 cells that were cultured in DMEM (Nacalai Tesque Inc., Low-Glucose) supplemented with 10% FBS (biowest), 1% penicillin-streptomycin mixed solution (Nacalai Tesque Inc., Stabilized), and 0.5 mM sodium pyruvate at 37°C under 5% CO₂ were seeded at a concentration of 7,500 cells/100 µL per well in DMEM containing 10% FBS in a 96-well plate, and cultured at 37°C under 5% CO₂ for 24 hours; subsequently, the antisense oligonucleotide synthesized in Example 1 was added to prepare a final concentration of 50 nM, 100 nM, or 200 nM, and cells were further cultured at 37°C under 5% CO₂ for 24 hours. For extraction of RNA from the transfected cells, RNA was extracted and reverse-transcribed into cDNA using Cell Lysis & RT Kit (TOYOBO SuperPrep^{™}Cell Lysis & RT Kit for qPCR).

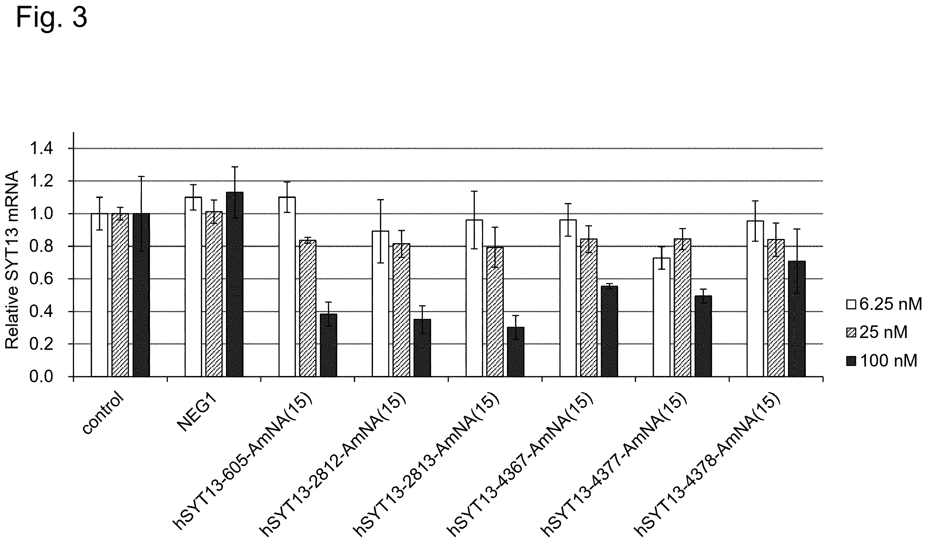

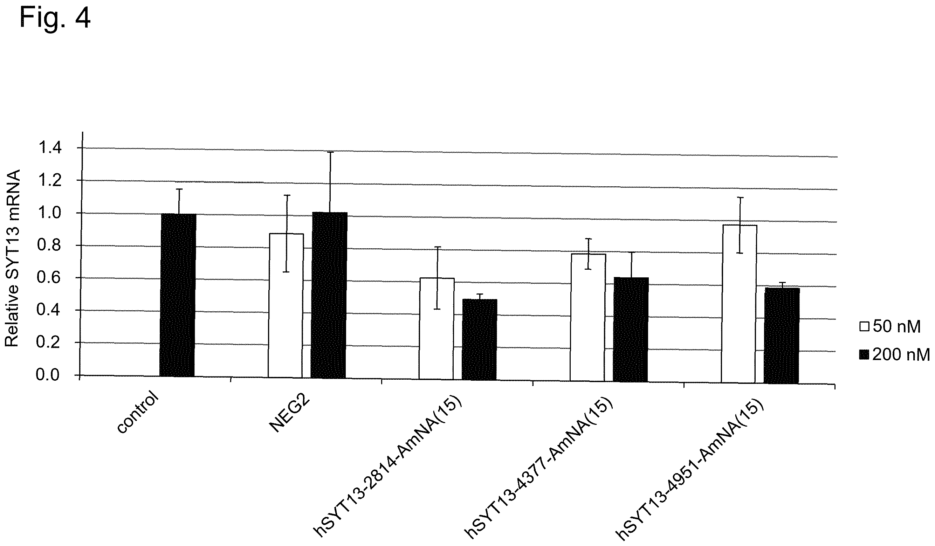

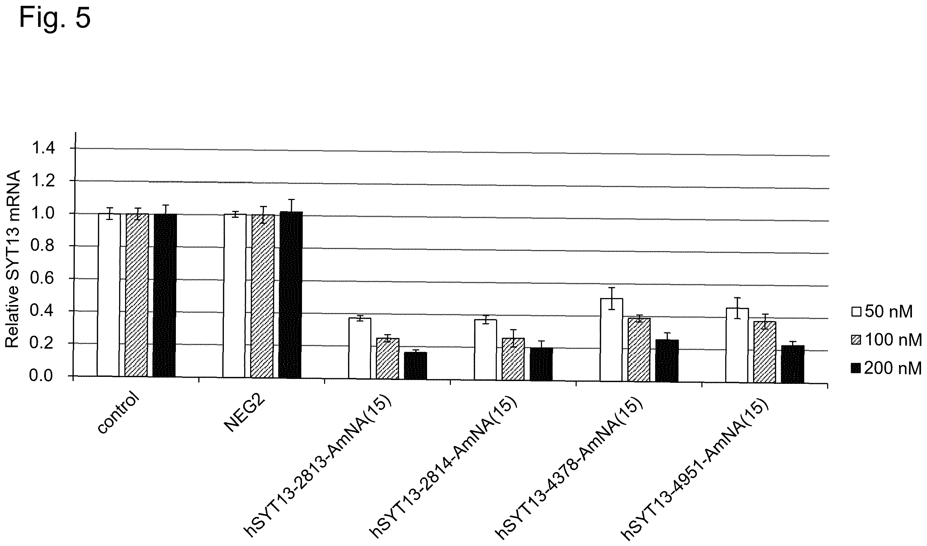

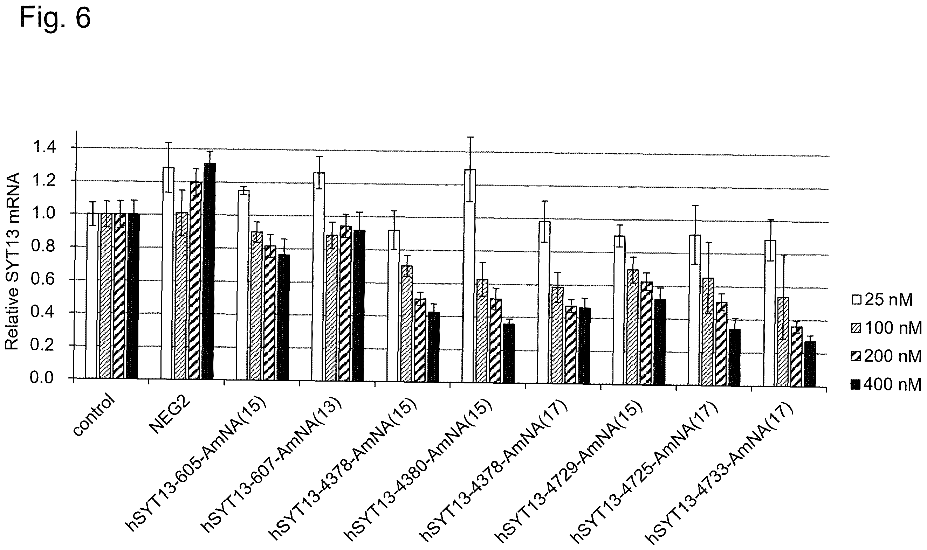

As a result, as shown in Figures 3 to 6, the concentration-dependent expression inhibition by the antisense oligonucleotide of the present invention was demonstrated for all cell lines.

### [Example 4 Examination for optimization of antisense oligonucleotide 1]

For each of the three antisense oligonucleotides, hSYT13-605-AmNA (15), hSYT113-4378-AmNA (15), and hSYT13-4729-AmNA (15), which were confirmed to have particularly good efficacy in the screening test of Example 2, ten antisense oligonucleotides were designed and synthesized by varying the length and position of the native nucleic acid region with the number of introduced artificial nucleic acids fixed. Table 2 below shows sequence information for antisense oligonucleotides designed for the optimization examination using hSYT13-605-AmNA (15) as a parent sequence, Table 3 shows sequence information for antisense oligonucleotides designed for the optimization examination using hSYT113-4378-AmNA (15) as a parent sequence, and Table 4 shows sequence information for antisense oligonucleotides designed for the optimization examination using hSYT13-4729-AmNA (15) as a parent sequence

**[Table 2]**

| Examination for optimization of hSYT13-605-AmNA (15) | | |
|---|---|---|
| Sequence Name | Antisense (5'→3') | SEQ ID NO: |
| hSYT13-605-AmNA(15) | A(Y)^G(Y)^T(Y)^a^g^t^g^g^a^g^t^t^T(Y)^G(Y)^g | 4 |
| hSYT13-607-AmNA(13) | A(Y)^G(Y)^T(Y)^a^g^t^g^g^a^g^T(Y)^T(Y)^t | 50 |
| hSYT13-609-AmNA(13) | G(Y)^5(Y)^A(Y)^g^t^a^g^t^g^g^A(Y)^G(Y)^t | 51 |
| hSYT13-603-AmNA(15) | T(Y)^A(Y)^G(Y)^t^g^g^a^g^t^t^t^g^G(Y) ^G(Y)^g | 52 |
| hSYT13-607-AmNA(15) | G(Y)^5(Y)^A(Y)^g^t^a^g^t^g^g^a^g^T(Y)^T(Y)^t | 53 |
| hSYT13-609-AmNA(15) | A(Y)^G(Y)^G(Y)^c^a^g^t^a^g^t^g^g^A(Y)^G(Y)^t | 54 |
| hSYT13-601-AmNA(17) | T(Y)^A(Y)^G(Y)^t^g^g^a^g^t^t^t^g^g^g^G(Y)^G(Y)^c | 55 |
| hSYT13-603-AmNA(17) | A(Y)^G(Y)^T(Y)^a^g^t^g^g^a^g^t^t^t^g^G(Y)^G(Y)^g | 56 |
| hSYT13-605-AmNA(17) | G(Y)^5(Y)^A(Y)^g^t^a^g^t^g^g^a^g^t^t^T(Y)^G(Y)^g | 57 |
| hSYT13-607-AmNA(17) | A(Y)^G(Y)^G(Y)^c^a^g^t^a^g^t^g^g^a^g^T(Y)^T(Y)^t | 58 |
| hSYT13-609-AmNA(17) | 5(Y)^5(Y)^A(Y)^g^g^c^a^g^t^a^g^t^g^g^A(Y)^G(Y)^t | 59 |

| | | |
|---|---|---|
| A(Y), G(Y), 5(Y), T(Y) = AmNA a, g, t, c = DNA ^ = Phosphorothioate bond 5 = mC | | |

**[Table 3]**

| Examination for optimization of hSYT113-4378-AmNA (15) | | |
|---|---|---|
| Sequence Name | Antisense (5'→3') | SEQ ID NO: |
| hSYT13-4378-AmNA(15) | T(Y)^5(Y)^T(Y)^c^t^t^c^a^t^c^t^g^T(Y)^T(Y)^g | 35 |
| hSYT13-4374-AmNA(13) | 5(Y)^A(Y)^T(Y)^c^t^g^t^t^g^a^T(Y)^T(Y)^a | 60 |
| hSYT13-4376-AmNA(15) | T(Y)^5(Y)^T(Y)^t^c^a^t^c^t^g^t^t^G(Y)^A(Y)^t | 61 |
| hSYT13-4380-AmNA(15) | A(Y)^T(Y)^T(Y)^c^t^c^t^t^c^a^t^c^T(Y)^G(Y)^t | 62 |
| hSYT13-4382-AmNA(15) | A(Y)^T(Y)^A(Y)^t^t^c^t^c^t^t^c^a^T(Y)^5(Y)^t | 63 |
| hSYT13-4374-AmNA(17) | T(Y)^5(Y)^T(Y)^t^c^a^t^c^t^g^t^t^g^a^T(Y)^T(Y)^a | 64 |
| hSYT13-4376-AmNA(17) | T(Y)^5(Y)^T(Y)^c^t^t^c^a^t^c^t^g^t^t^G(Y)^A(Y)^t | 65 |
| hSYT13-4378-AmNA(17) | A(Y)^T(Y)^T(Y)^c^t^c^t^t^c^a^t^c^t^g^T(Y)^T(Y)^g | 66 |
| hSYT13-4380-AmNA(17) | A(Y)^T(Y)^A(Y)^t^t^c^t^c^t^t^c^a^t^c^T(Y)^G(Y)^t | 67 |
| hSYT13-4382-AmNA(17) | T(Y)^T(Y)^A(Y)^t^a^t^t^c^t^c^t^t^c^a^T(Y)^5(Y)^t | 68 |
| hSYT13-4374-AmNA(19) | T(Y)^5(Y)^T(Y)^c^t^t^c^a^t^c^t^g^t^t^g^a^T(Y)^T(Y)^a | 69 |

| | | |
|---|---|---|
| A(Y), G(Y), 5(Y), T(Y) = AmNA a, g, t, c = DNA ^ = Phosphorothioate bond 5 = mC | | |

**[Table 4]**

| Examination for optimization of hSYT13-4729-AmNA (15) | | |
|---|---|---|
| Sequence Name | Antisense (5'→3') | SEQ ID NO: |
| hSYT13-4729-AmNA(15) | A(Y)^T(Y)^G(Y)^a^g^g^g^c^a^g^a^c^T(Y)^5(Y)^c | 39 |
| hSYT13-4725-AmNA(13) | G(Y)^5(Y)^A(Y)^g^a^c^t^c^c^c^G(Y)^A(Y)^t | 70 |
| hSYT13-4727-AmNA(13) | G(Y)^G(Y)^G(Y)^c^a^g^a^c^t^c^5(Y)^5(Y)^g | 71 |
| hSYT13-4725-AmNA(15) | G(Y)^G(Y)^G(Y)^c^a^g^a^c^t^c^c^c^G(Y)^A(Y)^t | 72 |
| hSYT13-4727-AmNA(15) | G(Y)^A(Y)^G(Y)^g^g^c^a^g^a^c^t^c^5(Y)^5(Y)^g | 73 |
| hSYT13-4731-AmNA(15) | T(Y)^A(Y)^A(Y)^t^g^a^g^g^g^c^a^g^A(Y)^5(Y)^t | 74 |
| hSYT13-4725-AmNA(17) | G(Y)^A(Y)^G(Y)^g^g^c^a^g^a^c^t^c^c^c^G(Y)^A(Y)^t | 75 |
| hSYT13-4727-AmNA(17) | A(Y)^T(Y)^G(Y)^a^g^g^g^c^a^g^a^c^t^c^5(Y)^5(Y)^g | 76 |
| hSYT13-4729-AmNA(17) | T(Y)^A(Y)^A(Y)^t^g^a^g^g^g^c^a^g^a^c^T(Y)^5(Y)^c | 77 |
| hSYT13-4731-AmNA(17) | A(Y)^T(Y)^T(Y)^a^a^t^g^a^g^g^g^c^a^g^A(Y)^5(Y)^t | 78 |
| hSYT13-4733-AmNA(17) | A(Y)^G(Y)^A(Y)^t^t^a^a^t^g^a^g^g^g^c^A(Y)^G(Y)^a | 79 |

| | | |
|---|---|---|
| A(Y), G(Y), 5(Y), T(Y) = AmNA a, g, t, c = DNA ^ = Phosphorothioate bond 5 = mC | | |

### [Example 5 Inhibitory effect on SYT13 mRNA expression in cancer cells]

Each of the antisense oligonucleotides synthesized in Example 4 was examined for the inhibitory effect on the expression of SYT13 in OCUM-1, MKN1, and NUGC-4 cells in the same manner as in Example 3. In this Example, the final concentration of antisense oligonucleotides was set to 100 nM or 400 nM.

The operational procedures for the OCUM-1 and MKN1 cells were the same as in Example 3.

For NUGC-4 cells, NUGC-4 cells that were cultured in RPMI 1640 (Nacalai Tesque Inc.) supplemented with 10% FBS (biowest) and 1% penicillin-streptomycin mixed solution (Nacalai Tesque Inc., Stabilized) at 37°C under 5% CO₂ were seeded at a concentration of 8,000 cells/100 µL per well in DMEM containing 10% FBS in a 96-well plate, and cultured at 37°C under 5% CO₂ for 24 hours. For extraction of RNA from the transfected cells, RNA was extracted and reverse-transcribed into cDNA using Cell Lysis & RT Kit (TOYOBO SuperPrep^{™}Cell Lysis & RT Kit for qPCR).

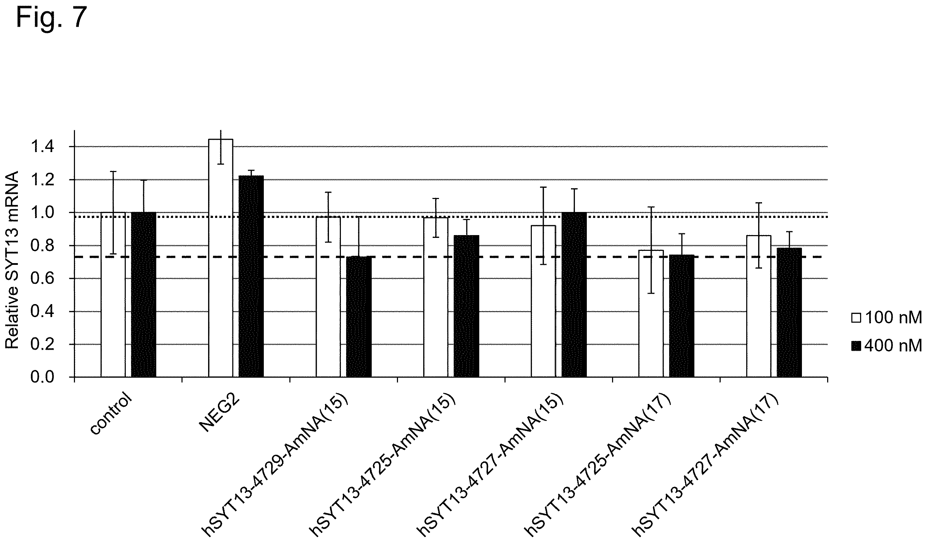

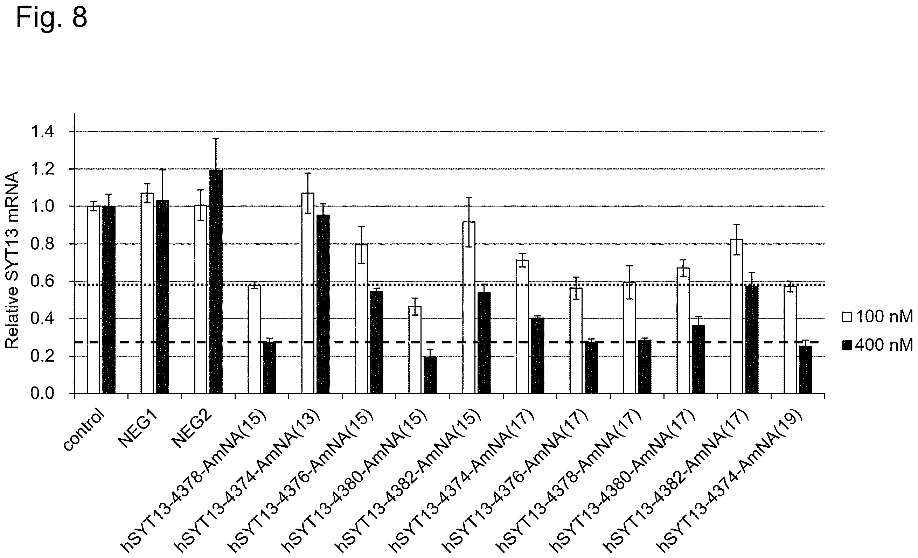

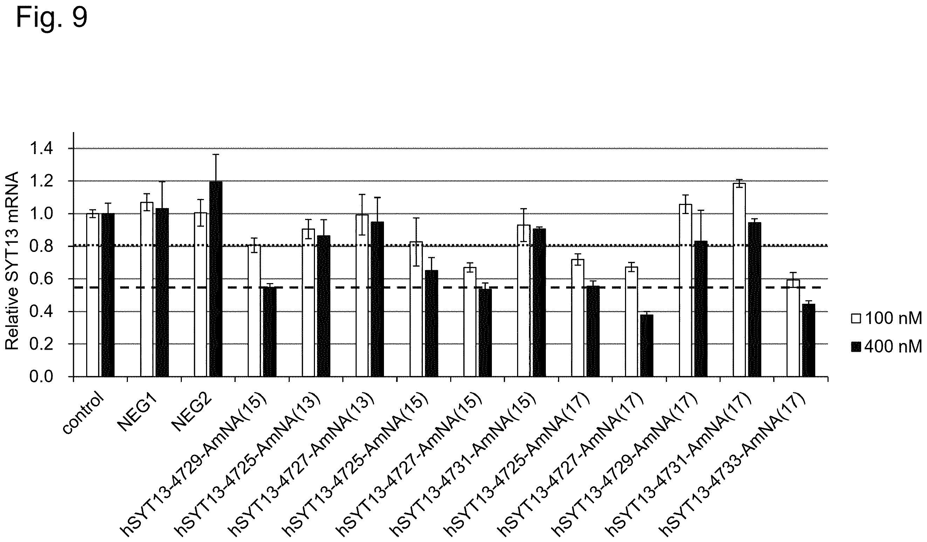

As a result, as shown in Figures 7 to 9, some of the antisense oligonucleotides designed for optimization with hSYT13-4729-AmNA (15) and hSYT113-4378-AmNA (15) as parent sequences were found to have higher inhibitory activity on the expression of SYT13 mRNA than the parent sequences.

### [Example 6 Inhibitory effect 1 on proliferative capacity]

hSYT13-605-AmNA (15), hSYT13-2813-AmNA (15), hSYT13-4367-AmNA (15), hSYT13-4378-AmNA (15), and hSYT13-4729-AmNA (15), the sequence information of which was shown in Table 1, and which were shown to have high inhibitory effect on the expression of SYT13 mRNA as confirmed in Example 2, were examined for an inhibitory effect on the proliferative capacity of cancer cells *in vitro.*

MKN1/Luc, NUGC4, AGS, N87, and GSU cells were seeded at a concentration of 3000 cells/well and KATO3 and OCUM1 cells were seeded at a concentration of 5000 cells/well in a 96-well plate, transfected with the antisense oligonucleotide at a final concentration of 400 nM (MKN1/Luc, NUGC4) or 100 nM (AGS, N87, GSU, KATO3, OCUM1) by the CEM method and then cultured; the number of cells was determined using Cell Counting Kit-8 (Dojindo Molecular Technologies, Inc.) on days 0, 1, 3, and 5, and a fold change relative to the number of cells at the initial stage of culture was calculated. The fold changes in eight wells were determined for each sample, and their mean value and standard deviation were calculated.

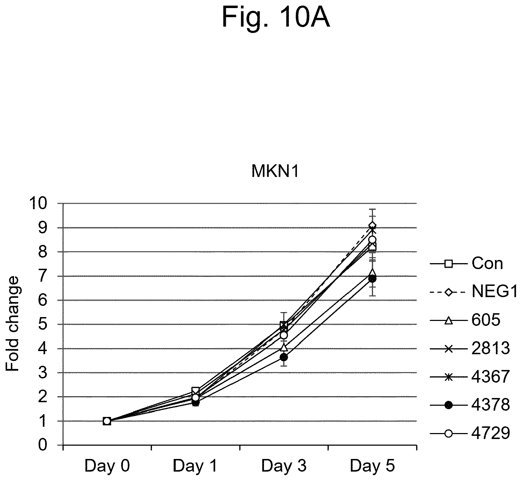

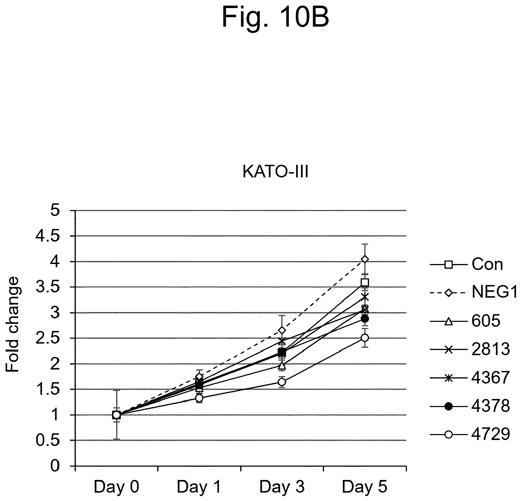

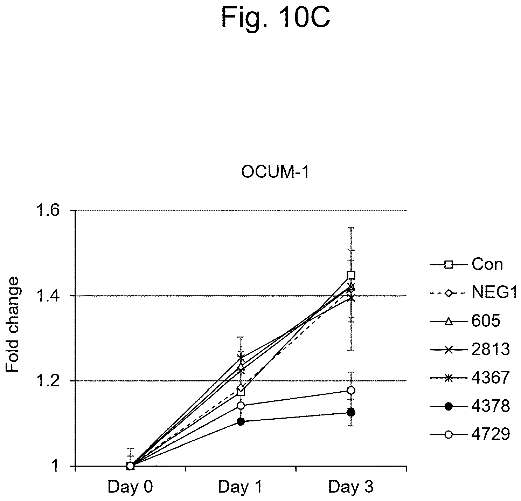

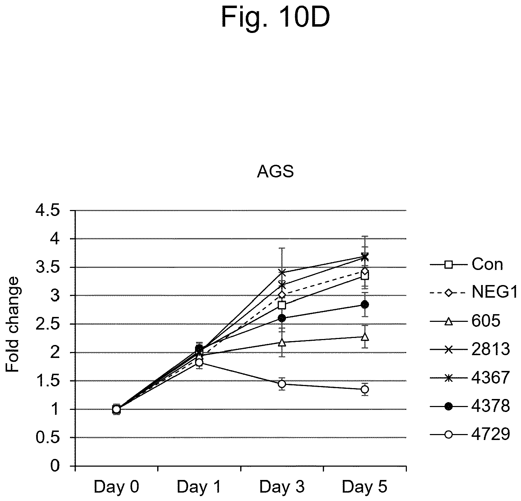

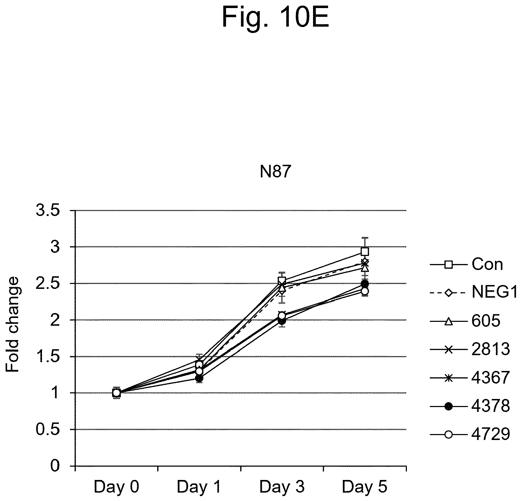

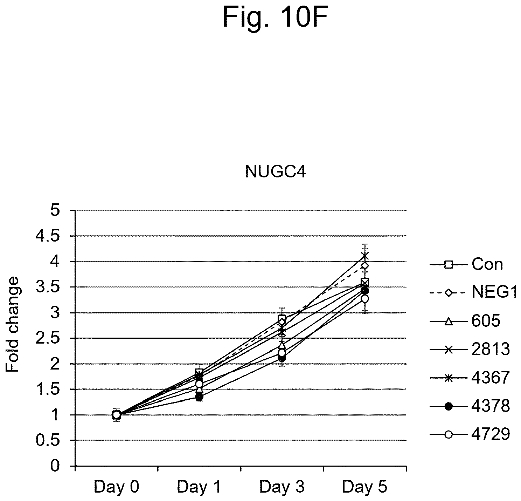

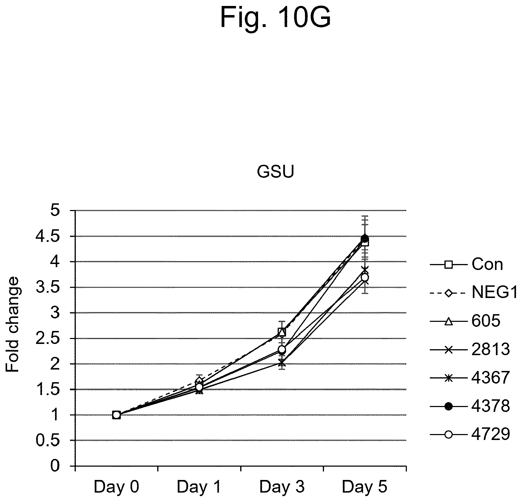

As a result, as shown in Figures 10A to 10G, it was shown that the above five antisense oligonucleotides have a significant inhibitory effect on the proliferation of the human gastric cancer cell lines, although the results varied depending on the cell lines used.

### [Reference Example Inhibitory effect on proliferation by siRNA]

Accell SYT13 siRNA (manufactured by Dharmacon Inc.) was used as siRNA that can target human SYT13 mRNA, and 400 nM of the siRNA was transfected into 50,000 cells/mL of MKN1 and NUGC4 cell lines by the CEM method in the same manner as in Example 6, and then, the effect on the proliferative capacity of these cell lines was examined. As a control, siRNA designed not to bind to any of the known genes (Accell Green Non-targeting, manufactured by Dharmacon Inc.) was used for comparison.

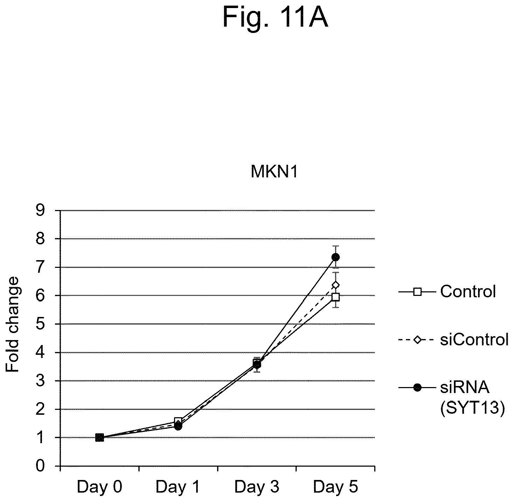

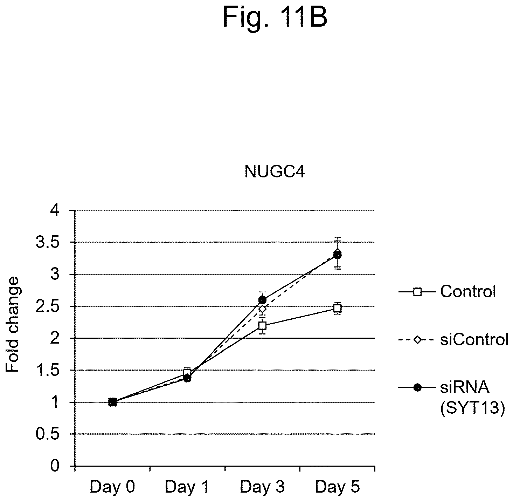

As a result, as shown in Figures 11A and 11B, the use of 400 nM of the siRNA attained no significant inhibitory effect on the proliferation as compared to the control for either of MKN1 and NUGC4 cell lines.

### [Example 7 Inhibitory effect on migration capacity 1]

An ibidi Culture-Insert (ibidi GmbH, Martinsried, Germany) was used to examine the inhibitory effect of the antisense oligonucleotide of the present invention on the migration capacity of gastric cancer cell lines *in vitro.*

Using MKN1/Luc (3 × 10⁴ cells/well), N87 (30 × 10⁴ cells/well), and NUGC4 (3.5 × 10⁴ cells/well) as the cells as well as any one of antisense oligonucleotides hSYT13-605-AmNA (15), hSYT13-2813-AmNA (15), hSYT13-4367-AmNA (15), hSYT13-4378-AmNA (15), and hSYT13-4729-AmNA (15) of the present invention, or a control antisense oligonucleotide (NEG1), the inhibitory effect on the cell migration into a cell-free gap was examined.

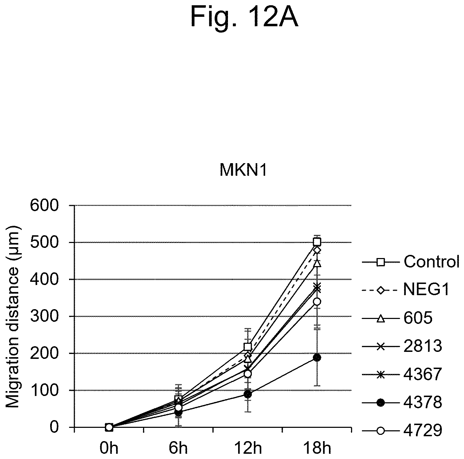

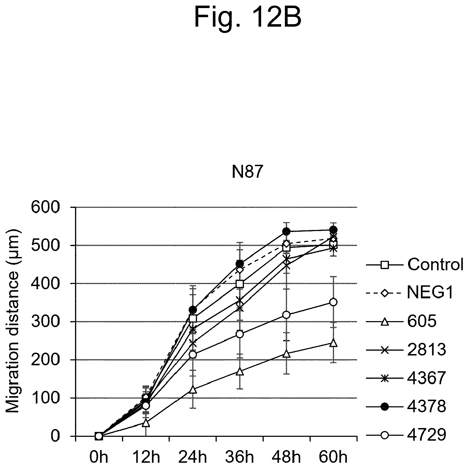

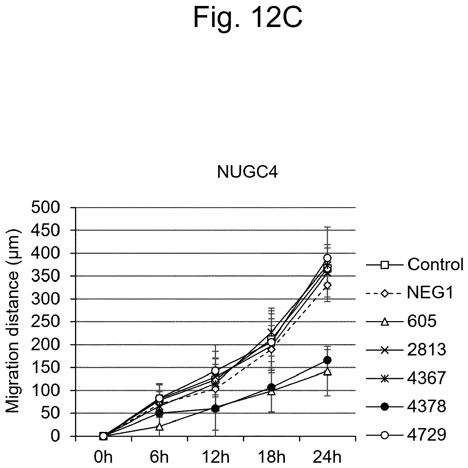

As a result, as shown in Figures 12A to 12C, it was shown that the above five antisense oligonucleotides have significant inhibitory effects on the migration of the human gastric cancer cell lines, although the results varied depending on the cell lines used.

### [Example 8 Inhibitory effect on invasive capacity 1]

BioCoat Matrigel Invasion Chamber (BD Biosciences, Bedford, MA, USA) was used to examine the inhibitory effect of the antisense oligonucleotide of the present invention on the invasive capacity of gastric cancer cell lines *in vitro.*

Using MKN1/Luc (2.5 × 10⁴ cells/well), AGS (5 × 10⁴ cells/well), GSU (5 × 10⁴ cells/well) as the cells as well as any one of antisense oligonucleotides hSYT13-605-AmNA (15), hSYT13-2813-AmNA (15), hSYT13-4367-AmNA (15), hSYT13-4378-AmNA (15), and hSYT13-4729-AmNA (15) of the present invention, or a control antisense oligonucleotide (NEG1), the numbers of invasive cells in the chamber were compared.

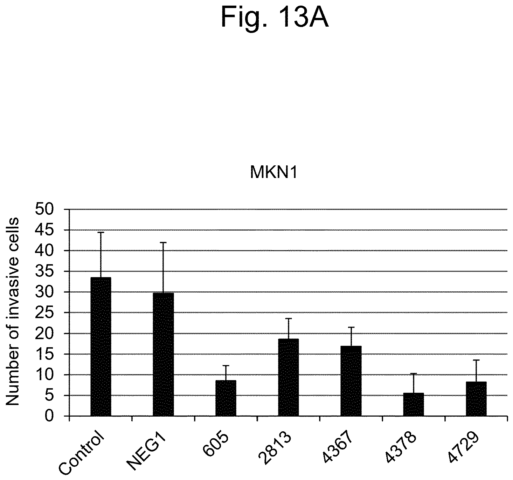

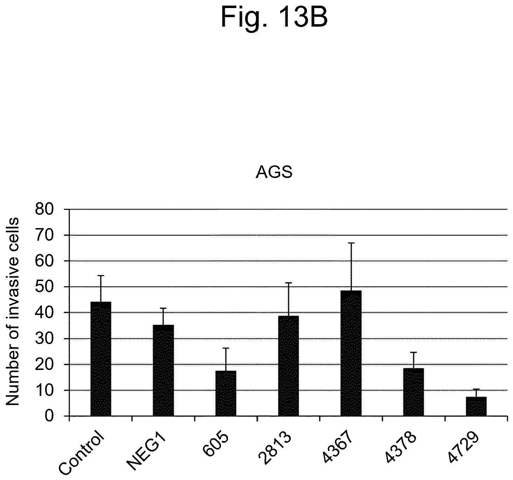

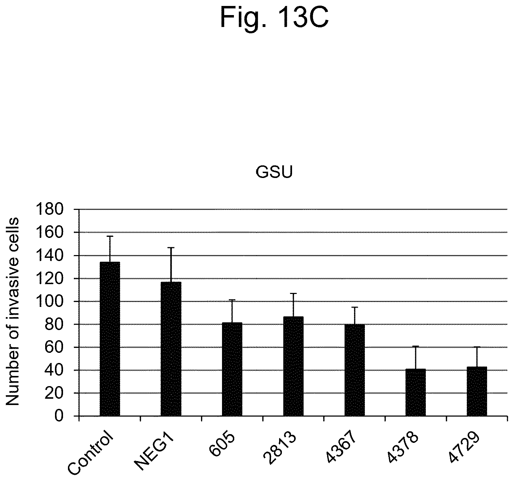

As a result, as shown in Figures 13A to 13C, it was shown that the above five antisense oligonucleotides have significant inhibitory effects on the invasion of the human gastric cancer cell lines, although the results varied depending on the cell lines used.

### [Example 9 In vivo test 1]

Using the same method as that described in WO2016/143697, immunodeficient mice (10-week-old male BALBc-nu/nu) were intraperitoneally administered with 1 ml of luciferasegene-introduced MKN1-luc cells or NUGC4 cells at a dose of 1 × 10⁶ cells/ml to prepare a peritoneal dissemination model, and the *in vivo* efficacy of the antisense oligonucleotide of the present invention was examined.

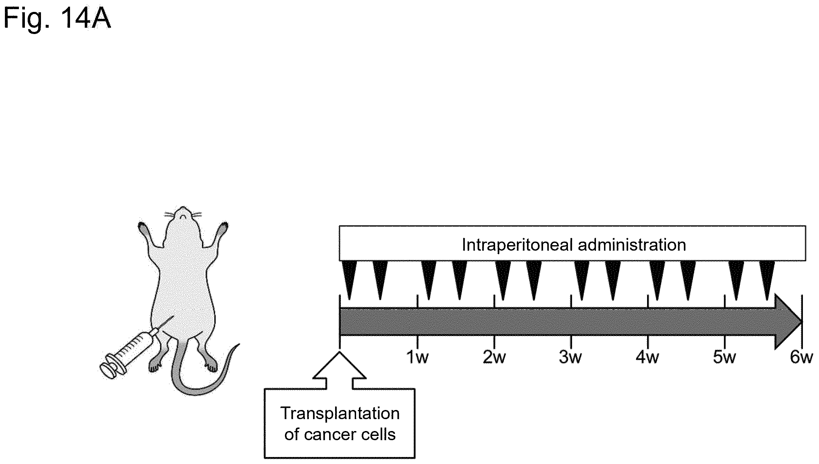

After the transplantation of the cancer cells, 0.2 mg per dose (equivalent to 10 mg/kg with a mouse body weight of 20 g) of hSYT13-4729 (15) or hSYT13-4378 (15) (both with a molecular weight of about 5000) added to 500 µL of 5% glucose solution was administered twice a week for 6 weeks (Figure 14A). As a control, 0.2 mg per dose (equivalent to 10 mg/kg with a mouse body weight of 20 g) of a control antisense oligonucleotide (NEG1, SEQ ID NO: 44) or Accell SYT13 siRNA (manufactured by Dharmacon Inc.) used in the above Reference Example was administered for 6 weeks.

The mice transplanted with MKN1-luc cells were subjected to *in vivo* imaging using an *In Vivo* Imaging System (IVIS^{®}) Lumina (Xenogen Corporation, Alameda, California, USA). Specifically, 2, 4, or 6 weeks after the cell transplantation, the mice were intraperitoneally administered with D-luciferin (150 mg/kg) (Summit Pharmaceuticals International, Tokyo, Japan), and 15 minutes later, photographed with IVIS^{®} to measure a signal intensity with Living Image^{®} Version 2.6 Software (Xenogen Corporation). As a result, in the mice to which the antisense oligonucleotide was not administered and the mice to which the control antisense oligonucleotide or siRNA was administered, luminescence indicating the survival of peritoneal dissemination-like cells was observed, whereas in the mice to which the antisense oligonucleotide of the present invention was administered, luminescence could not be detected, or even if it could, the amount of luminescence was significantly small (data not shown).

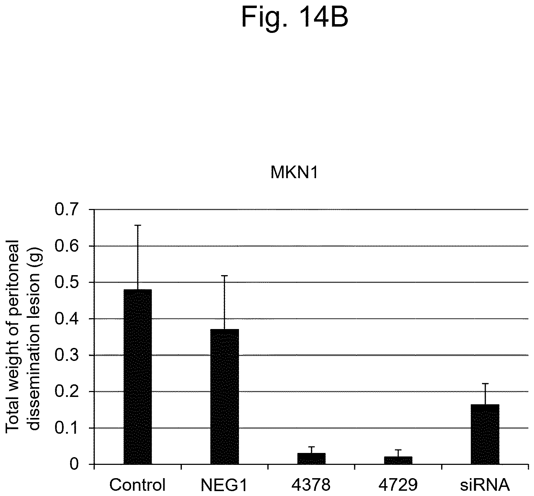

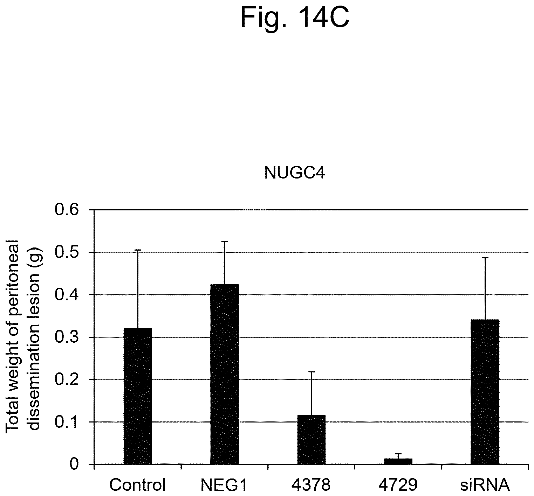

Six weeks after the cancer cell transplantation, some mice were sacrificed to compare total weights of peritoneal dissemination lesions of the mice for each group. The results are shown in Figures 14B and 14C. Compared with the mice with no antisense oligonucleotide administration, the mice with the control antisense oligonucleotide administration, and the mice with siRNA administration, the mice with the antisense oligonucleotide of the present invention administration (hSYT13-4729 (15) and hSYT13-4378 (15)) had significantly lower total weight of peritoneal dissemination lesion, which demonstrates that the antisense oligonucleotide of the present invention can effectively inhibit peritoneal dissemination.

### [Example 10 Inhibitory effect on proliferative capacity 2]

Four antisense oligonucleotides, hSYT13-4380 (17) and hSYT13-4733 (17) (for which sequence information is shown in Table 3 and Table 4, respectively), which showed suitable activity in Example 5, in addition to hSYT13-4378 (15) and hSYT13-4729 (15) were used to examine the inhibitory effect on the proliferative capacity of MKN1 cells or NUGC4 cells *in vitro* in the same manner as in Example 6.

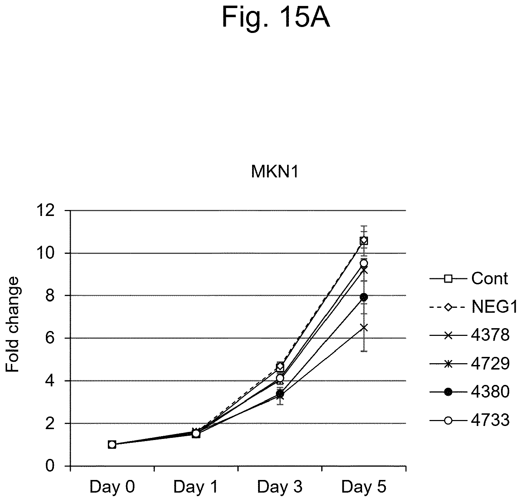

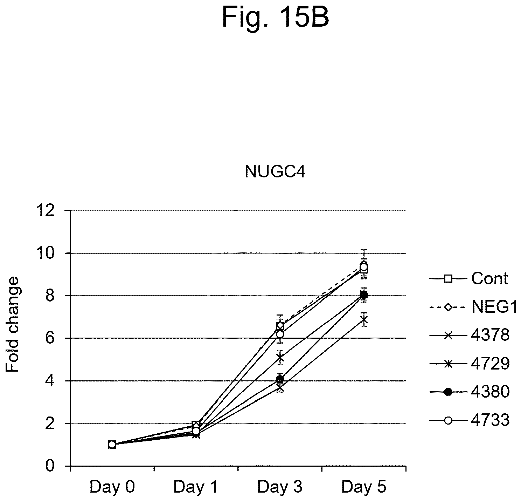

As a result, as shown in Figures 15A and 15B, significant inhibitory effects on proliferation were observed in hSYT13-4378 (15), hSYT13-4729 (15), and hSYT13-4380 (17) as compared with the group with no antisense oligonucleotide added (Cont) and the group with the control antisense oligonucleotide added (NEG1), under the tested conditions.

### [Example 11 Inhibitory effect on migration capacity 2]

Four antisense oligonucleotides, hSYT13-4378 (15), hSYT13-4729 (15), hSYT13-4380 (17), and hSYT13-4733 (17) were used to examine the inhibitory effect on the migration capacity of MKN1 cells or NUGC4 cells *in vitro* in the same manner as in Example 7.

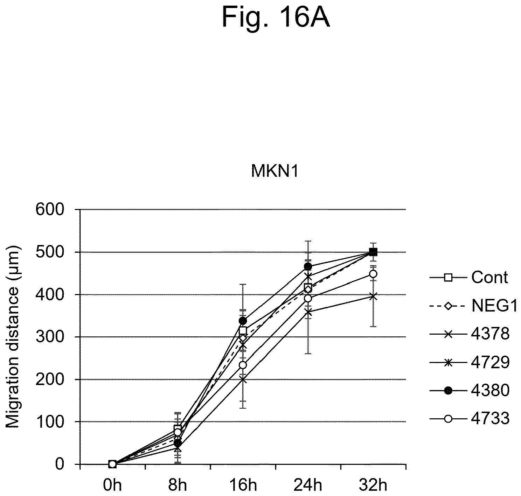

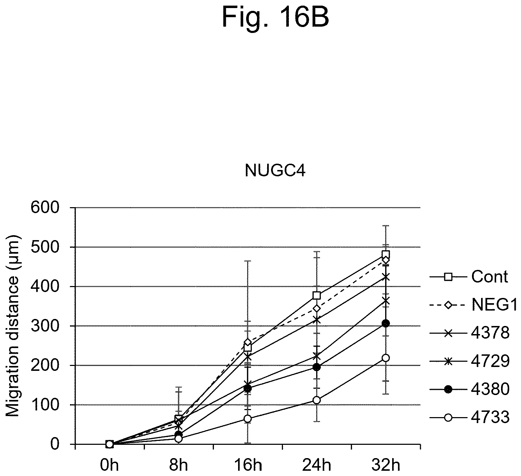

As a result, as shown in Figures 16A and 16B, significant inhibitory effects on migration were observed in hSYT13-4378 (15), hSYT13-4380 (17), and hSYT13-4733 (17) as compared with the group with no antisense oligonucleotide added (Cont) and the group with the control antisense oligonucleotide added (NEG1), under the tested conditions.

### [Example 12 Inhibitory effect on invasive capacity 2]

Four antisense oligonucleotides, hSYT13-4378 (15), hSYT13-4729 (15), hSYT13-4380 (17), and hSYT13-4733 (17) were used to examine the inhibitory effect on the invasive capacity of MKN1 cells or NUGC4 cells *in vitro* in the same manner as in Example 8.

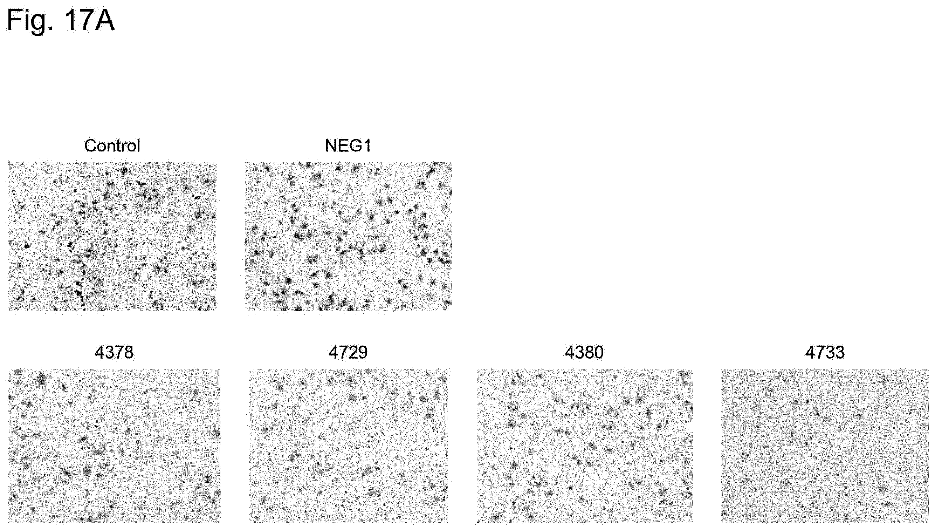

As a result, as shown in Figure 17A, it was shown that the invasive capacity was inhibited when the antisense oligonucleotide of the present invention was added, as compared with the group with no antisense oligonucleotide added (Cont) and the group with the control antisense oligonucleotide added (NEG1).

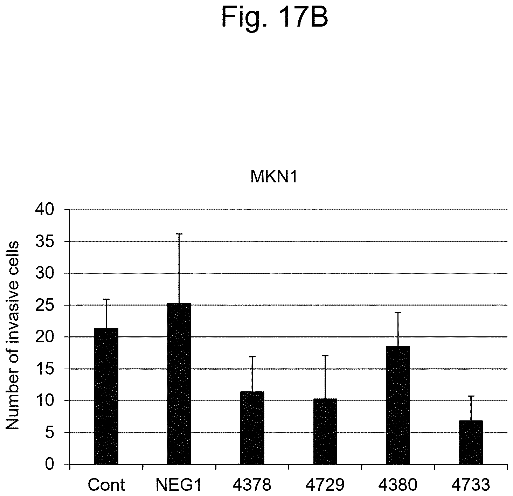

As shown in Figure 17B, in which the numbers of invasive cells are compared, significant inhibitory effects on invasion was observed in hSYT13-4378 (15), hSYT13-4729 (15), and hSYT13-4733 (17) as compared with the group with no antisense oligonucleotide added (Cont) and the group with the control antisense oligonucleotide added (NEG1), under the tested conditions.

### [Example 13 In vivo test 2]

An *in vivo* test was performed using a mouse model of peritoneal dissemination prepared in the same manner as in Example 9.

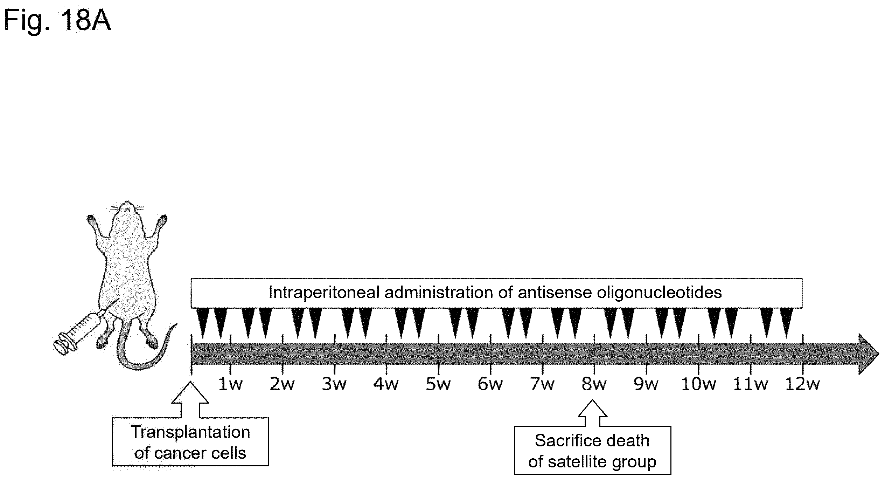

A mouse model of peritoneal dissemination was prepared by intraperitoneal implantation of 1 ml of NUGC4 cell line at 2 × 10⁶ cells/ml, and thereafter, 0.2 mg per dose (equivalent to 10 mg/kg with a mouse body weight of 20 g) of antisense oligonucleotides hSYT13-4378 (15) and hSYT13-4733 (17), and a control antisense oligonucleotide (NEG1, SEQ ID NO: 44) added to 500 µL of 5% glucose solution were administered twice a week for 12 weeks (Figure 18A).

Eight weeks after the transplantation of the cancer cells, some mice were sacrificed for macroscopic examination on tumor proliferation. As a result, the mice with no antisense oligonucleotide administration (Control) and the mice with the control antisense oligonucleotide administration (NEG1) showed a significant growth of peritoneal dissemination-like tumor, whereas the mice with hSYT13-4378 (15) administration and the mice with hSYT13-4733 (17) administration had almost no peritoneal dissemination confirmed (data not shown).

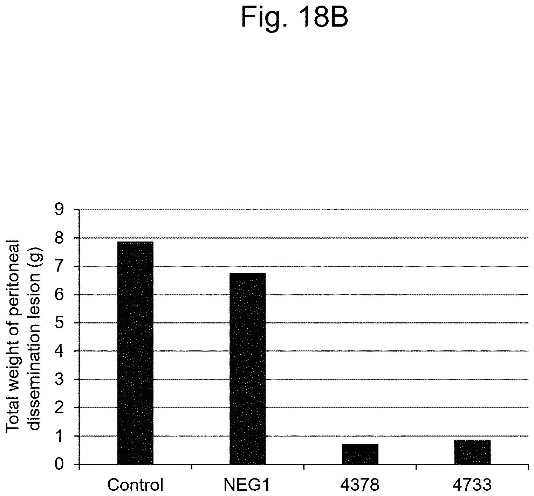

Figure 18B shows a comparison of the total weights of peritoneal dissemination lesions of the mice for each group. As confirmed through the microscopic examination, the mice with the antisense oligonucleotide of the present invention administration had a quite small total weight of peritoneal dissemination lesion as compared with the control mice (the mice with no antisense oligonucleotide administration, and the mice with the control antisense oligonucleotide administration), which demonstrates that the antisense oligonucleotide of the present invention can effectively inhibit peritoneal dissemination.

### [Example 14 Survival analysis]

The number of days of survival after cancer cell transplantation (seeding) was determined for mice with and without the antisense oligonucleotide administration for 12 weeks (8 mice per group) in Example 13.

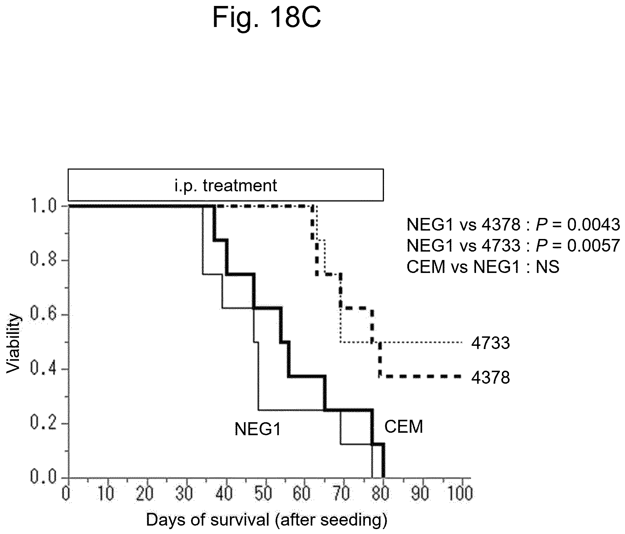

As a result, as shown in Figure 18C, it was shown that the mice with the antisense oligonucleotide of the present invention administration had significantly longer survival than the group with no antisense oligonucleotide administration (CEM) and the group with the control antisense oligonucleotide administration (NEG1), which demonstrates that the antisense oligonucleotide of the present invention has an inhibitory effect against recurrence due to peritoneal dissemination.

### [Example 15 Demonstration of inhibitory effects on SYT13 mRNA expression using antisense oligonucleotides with different modifications]

Based on hSYT13-4733-AmNA (17) (SEQ ID NO: 79), which was shown to have high inhibitory activity on the expression of SYT13 mRNA in Example 5, fourteen antisense oligonucleotides (4733-A to 4733-N), with its modification patterns of sugar, nucleobase and/or internucleoside linkage changed, were designed and synthesized. The sequence information for these antisense oligonucleotides is shown in Table 5.

**[Table 5]**

| Examination of modification patterns based on hSYT13-4733-AmNA (17) | | |
|---|---|---|
| Sequence Name | Antisense (5'→3') | SEQ ID NO: |
| hSYT13-4733-AmNA(17) | A(Y)^G(Y)^A(Y)^t^t^a^a^t^g^a^g^g^g^c^A(Y)^G(Y)^a | 79 |
| 4733-A | A(Y)^G(Y)A(Y)^t^t^a^a^t^g^a^g^g^g^c^A(Y)G(Y)^a | 80 |
| 4733-B | A(Y)^G(Y)A(Y)^t^t^a^a^t^g^a^g^g^g^c^A(Y)^G(Y)^a | 81 |
| 4733-C | A(Y)^G(Y)^A(Y)^t^t^d^3^t^g^a^g^g^g^c^A(Y)G(Y)^a | 82 |
| 4733-D | A(Y)^G(Y)^A(Y)t^t^a^a^t^g^a^g^g^g^c^A(Y)^G(Y)^a | 83 |
| 4733-E | A(Y)^G(Y)A(Y)t^t^a^a^t^g^a^g^g^g^c^A(Y)^G(Y)^a | 84 |
| 4733-F | A(Y)^G(Y)^A(Y)t^t^a^a^t^g^a^g^g^g^c^A(Y)G(Y)^a | 85 |
| 4733-G | A(Y)^g^A(Y)^t^t^a^a^t^g^a^g^g^g^c^A(Y)^G(Y)^a | 86 |
| 4733-H | A(Y)^g^A(Y)^t^t^a^a^t^g^a^g^g^g^5(Y)^a^G(Y)^a | 87 |
| 4733-I | A(Y)^g^A(Y)^t^t^a^a^t^g^a^g^g^g^c^a^G(Y)^a | 88 |
| 4733-J | A(Y)^g^a^T(Y)^t^a^a^t^g^a^g^g^g^c^A(Y)^G(Y)^a | 89 |
| 4733-K | A(Y)^g^a^T(Y)^t^a^a^t^g^a^g^g^g^5(Y)^a^G(Y)^a | 90 |
| 4733-L | A(Y)^g^a^T(Y)^t^a^a^t^g^a^g^g^g^c^a^G(Y)^a | 91 |
| 4733-M | A(Y)^G(Y)^A(Y)^t^t^a^a^t^g^a^g^g^g^5(Y)^a^G(Y)^a | 92 |
| 4733-N | A(Y)^G(Y)^A(Y)^t^t^a^a^t^g^a^g^g^g^c^a^G(Y)^a | 93 |

| | | |
|---|---|---|
| A(Y), G(Y), 5(Y), T(Y) = AmNA a, g, t, c = DNA ^ = Phosphorothioate bond 5 = mC | | |

As understood from Table 5 and the accompanying sequence listing, hSYT13-4733-AmNA (17) and 4733-A to 4733-N are gapmers that are 17 bases in length.

hSYT13-4733-AmNA (17) comprises non-native nucleosides (depicted as "AmNA" in the table) with a sugar having an amide bridge, three on the 5' end and two on the 3' end, and all the internucleoside linkages are phosphorothioate bonds.

In contrast, in six antisense oligonucleotides 4733-A to 4733-F, part of their internucleoside linkages in either or both of the wing regions at the 5' and 3' ends (5' wing region and 3' wing region) as well as at the boundary between the wing region and the DNA gap region are phosphodiester bonds.

In addition, in eight antisense oligonucleotides 4733-G to 4733-N, the number and/or position of non-native nucleosides (AmNAs) with a sugar having an amide bridge is changed in either or both of the 5' wing region and the 3' wing region, and 4733-H, 4733-K, and 4733-M include 5-methylcytosine in the 3' wing region.

hSYT13-4733-AmNA (17) (SEQ ID NO: 79) and the above fourteen antisense oligonucleotides (4733-A to 4733-N) were examined for the inhibitory effect on the expression of SYT13 in NUGC-4 cells in the same manner as in Example 5.

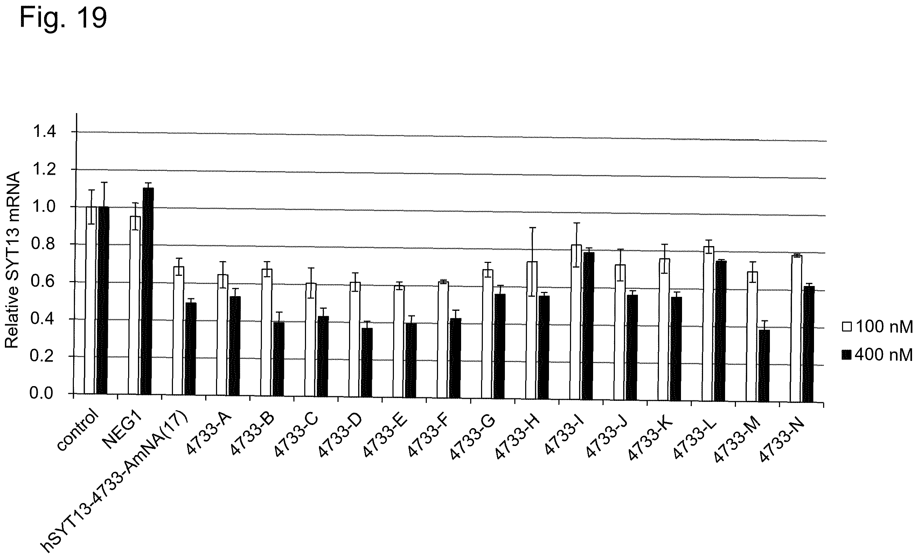

As a result, as shown in Figure 19, every antisense oligonucleotide significantly inhibited the expression of SYT13 mRNA *in vitro* as compared with the group with no antisense oligonucleotide addition (Control) and the group with the control antisense oligonucleotide addition (NEG1, the antisense oligonucleotide of SEQ ID NO: 44 added). In particular, 4733-B, 4733-C, 4733-D, 4733-E, 4733-F, and 4733-M were shown to have inhibitory effects on the expression that is comparable or superior to that of hSYT13-4733-AmNA (17), which demonstrates that they exhibit antisense activity suitable to inhibit the expression of SYT13.

All publications, patents, and patent applications cited herein are hereby incorporated by reference in their entirety.

## Claims

1. An antisense oligonucleotide capable of inhibiting expression of human SYT13 mRNA, the antisense oligonucleotide being:
an antisense oligonucleotide consisting of a 11- to 19-base-long nucleotide sequence complementary to a nucleotide sequence at positions 4714 to 4751 in a nucleotide sequence set forth in SEQ ID NO: 1; or
an antisense oligonucleotide consisting of a nucleotide sequence in which one to three bases, one to two bases, or one base is substituted, deleted, or inserted with regard to said antisense oligonucleotide.

2. An antisense oligonucleotide capable of inhibiting expression of human SYT13 mRNA, the antisense oligonucleotide being:
an antisense oligonucleotide consisting of a 11- to 19-base-long nucleotide sequence complementary to a nucleotide sequence at positions 348 to 366, 599 to 627, 997 to 1016, 1069 to 1088, 1419 to 1437, 1612 to 1641, 1775 to 1793, 2629 to 2647, 2810 to 2831, 3244 to 3262, 3315 to 3333, 3423 to 3442, 4266 to 4284, 4328 to 4346, 4365 to 4400, 4714 to 4751, 4776 to 4795, or 4949 to 4968 in a nucleotide sequence set forth in SEQ ID NO: 1; or
an antisense oligonucleotide consisting of a nucleotide sequence in which one to three bases, one to two bases, or one base is substituted, deleted, or inserted with regard to said antisense oligonucleotide.

3. An antisense oligonucleotide consisting of a nucleotide sequence selected from the group consisting of SEQ ID NOs: 3 to 43, or an antisense oligonucleotide consisting of a nucleotide sequence in which one to three bases are substituted, deleted, or inserted with regard to said antisense oligonucleotide.

4. An antisense oligonucleotide consisting of a nucleotide sequence selected from the group consisting of SEQ ID NOs: 50 to 59, or an antisense oligonucleotide consisting of a nucleotide sequence in which one to three bases are substituted, deleted, or inserted with regard to said antisense oligonucleotide.

5. An antisense oligonucleotide consisting of a nucleotide sequence selected from the group consisting of SEQ ID NOs: 60 to 69, or an antisense oligonucleotide consisting of a nucleotide sequence in which one to three bases are substituted, deleted, or inserted with regard to said antisense oligonucleotide.

6. An antisense oligonucleotide consisting of a nucleotide sequence selected from the group consisting of SEQ ID NOs: 70 to 79, or an antisense oligonucleotide consisting of a nucleotide sequence in which one to three bases are substituted, deleted, or inserted with regard to said antisense oligonucleotide.

7. An antisense oligonucleotide consisting of a nucleotide sequence selected from the group consisting of SEQ ID NOs: 4, 20, 29, 35, 39, 62, and 79, or an antisense oligonucleotide consisting of a nucleotide sequence in which one to three bases are substituted, deleted, or inserted with regard to said antisense oligonucleotide.

8. The antisense oligonucleotide according to any one of claims 1 to 7, wherein the antisense oligonucleotide has an artificial nucleic acid region containing a bicyclic sugar.

9. The antisense oligonucleotide according to any one of claims 1 to 8, wherein at least one internucleoside linkage is a phosphorothioate bond.

10. The antisense oligonucleotide according to any one of claims 1 to 9, wherein the antisense oligonucleotide has an artificial nucleic acid region containing 5-methylcytosine.

11. The antisense oligonucleotide according to any one of claims 1 to 10, wherein the antisense oligonucleotide is 15 to 19 nucleotides in length.

12. The antisense oligonucleotide according to any one of claims 1 to 11, wherein the antisense oligonucleotide is a gapmer.

13. A conjugate comprising: the antisense oligonucleotide according to any one of claims 1 to 12; and a further functional moiety directly or indirectly linked to the antisense oligonucleotide.

14. The conjugate according to claim 13, wherein the further functional moiety is a ligand for target molecule or an agent having antitumor activity.

15. A pharmaceutical composition comprising the antisense oligonucleotide according to any one of claims 1 to 12, or the conjugate according to claim 13 or 14.

16. The pharmaceutical composition according to claim 15, for treatment or prevention of gastric cancer in a human.

17. The pharmaceutical composition according to claim 16, for treatment or prevention of peritoneal dissemination after gastric cancer resection.


SEQUENCE LISTING

<110>  National University Corporation Tokai National Higher
       Education and Research System
       National Institutes of Biomedical Innovation, Health and
       Nutrition

<120>  Gastric Cancer Molecule Targeting Oligonucleotide Therapeutics

<130>  PH-8465-PCT

<150>  JP 2019-154968
<151>  2019-08-27

<160>  93

<170>  PatentIn version 3.5

<210>  1
<211>  5184
<212>  DNA
<213>  Homo sapiens

<400>  1
   gtccccgccc ctctggcggc gggagagctg ctggctcgcc cggatcccgg gagctgcctg      60
   gaggcgggcc cggcccgggg aaggtgagcg gctgcgggac ccagcccctc gccgggagcg     120
   ggcaccatgg tgctgtcggt gcctgtgatc gcgctgggcg ccacgctggg cacagccacc     180
   agcatcctcg cgttgtgcgg ggtcacctgc ctgtgtcggc acatgcaccc caagaagggg     240
   ctgctgccgc gggaccagga ccccgacctg gagaaggcga agcccagctt gctcgggtct     300
   gcacaacagt tcaatgttaa aaagtccacg gaacctgttc agccccgtgc cctcctcaag     360
   ttcccagaca tctatggacc caggccagct gtgacggctc cagaggtcat caactatgca     420
   gactattcac tgaggtctac ggaggagccc actgcacctg ccagccccca acccccgaat     480
   gacagtcgcc tcaagaggca ggtcacagag gagctgttca tcctccctca gaatggtgtg     540
   gtggaggatg tctgtgtcat ggagacctgg aacccagaga aggctgccag ttggaaccag     600
   gcccccaaac tccactactg cctggactat gactgtcaga aggcagaatt gtttgtgact     660
   cgcctggaag ctgtgaccag caaccacgac ggaggctgtg actgctacgt ccaagggagt      720
   gtggccaata ggaccggctc tgtggaggct cagacagccc taaagaagcg gcagctgcac     780
   accacctggg aggagggcct ggtgctcccc ctggcggagg aggagctccc cacagccacc     840
   ctgacgctga ccttgaggac ctgcgaccgc ttctcccgtc acagcgtggc cggggagctc     900
   cgcctgggcc tggacgggac atctgtgcct ctaggggctg cccagtgggg cgagctgaag     960
   acttcagcga aggagccatc tgcaggagct ggagaggtcc tactatccat cagctacctc    1020
   ccggctgcca accgcctcct ggtggtgctg attaaagcca agaacctcca ctctaaccag    1080
   tccaaggagc tcctggggaa ggatgtctct gtcaaggtga ccttgaagca ccaggctcgg    1140
   aagctgaaga agaagcagac taaacgagct aagcacaaga tcaaccccgt gtggaacgag    1200
   atgatcatgt ttgagctgcc tgacgacctg ctgcaggcct ccagtgtgga gctggaagtg 1260
   ctgggccagg acgattcagg gcagagctgt gcgcttggcc actgcagcct gggcctgcac 1320
   acctcgggct ctgagcgcag ccactgggag gagatgctca aaaaccctcg ccggcagatt 1380
   gccatgtggc accagctgca cctgtaacca gctgcccagc tgcctccctt cttggacagc 1440
   cctgacccgt cctctgcaac ctcctttctg tgcccctttc ctcattctga cacccagaag 1500
   acagtgacag atgtgtttgc aaggctggga tggctctctc atcatactct tgtttcttag 1560
   aaataagcaa gacagagcag gaaatggaat atgcgggtca cactgaggaa tgcattttgc 1620
   tcatctgtgt tattgaagga ggtgcttatt aaatacagtt cctatgcctg ttttataggt 1680
   ggggttaggc cagatgcaga gaaagctaaa tgtgggaatc atggatgcaa agaagaattt 1740
   ggctttttga aaaacaagca tttcaaaaat gatgaaggaa gtgaaagtat cctggatcaa 1800
   ctcctagagt tagagattgc ccaggtggaa agaaacctta gccagcgttc aatcaagctc 1860
   accatgcagg gcagtcaccc ggcagttctc aaactttagc atgtgaagag tcaccagcag 1920
   attcctgggc tcgcctggag acattcctag tcggtattcc tggtcgaagc ccaggagcct 1980
   tcctttttaa caagctgatg tagagggtgg agcactgtat gtggagaaat tccttctaca 2040
   atattccaca caggtttttg gccacagtcc ttgatggagt cccaaaacca tggtgcagcc 2100
   agttccaatg ctggacacct caaccatcag ggtgaaatct ggggcctcag ctttttaatt 2160
   taattatttt aattcttaat actttaattt gtgcatttca taagccccct gctcttggac 2220
   tgaattttgt gctttttatt gaagaatttt attgttttta tcttaaaatc agtttctatt 2280
   atccttgggg agaccatccc taacaaagta caggtgggat ctcctgtgag tcattggctg 2340
   ggttctgatt gctagatgtc acacccacca gcatcaccaa agtgactctg agatagaccg 2400
   gtcccttctc agcgttccag tcacttcagg aggaatttag ttattgactt agtctatgac 2460
   atctggctac atgtaggtag agaagaaaga taattttaaa aaggaaatca ggtcttttgc 2520
   aactgtgcct ccctctgtct gttttcactt gaatgggtaa ataaccagca gctaggtttt 2580
   gaattcctac cttgttattc taaacagatg tccacattgt taattaaatc taaattatga 2640
   gccttgctga gtggatacgg tacttacacc tgaaccagga ttcctgggtt ctgttgttga 2700
   cattgccctt cagcacctgt ttggccagct gtgtaagata ggactaatga ctaggaagcc 2760
   taccccaatg aatgatatac tagatgaaat agtgttcaaa acctgtaggc actctctggc 2820
   taaaaacaaa ctctgaggcc accagcagat catctttaag ctaagttact atttttcacc 2880
   ttttttttta gacggagttt tgctctttgt tgcccaggct ggagtgcagt ggcacgatct 2940
   cggctcactg caacctccgc ctcccaagtt caagcgattc tcctctctca gcctcctggg 3000
   tagctgggat tacaggtgcc caccaacatg cctggctaat ttttgtactt ttagtagaga 3060
   tggggtttca ccatgttggc caggctggtc ttcaactcca gatctcaggt gatctaccct 3120
   cctcggcctc ccaaagtact gggattacag gcctgagcca ccgcgcccgg cctatttttc 3180
   actttaattt ggcagctgag aatgcccaga aagtgccaga agcatcgtgg catttccaga 3240
   accatggatt ctgcctttgg acccctctct attaatatta aaactctggg ccttcagatg 3300
   tcaccctaat ccacttccct aagacagaat ttctggacaa gatgggtaag ggcttcattc 3360
   cttcaacaag tcaagtcata cttggcctct ccctgagaat ctgagcagga gccttataac 3420
   ctgtggtcat tattttttct ttctgtacag aaatagaaaa gcattagaaa taacttctaa 3480
   ccatcctctg aaaaaacaga aaaaatatcg aatccctctt tcatgagaag tcttttggat 3540
   aattggaaac cttcatcact gaggttggcc agcccctgcc aagtgttgtg taggcaaagc 3600
   acttgttagt ggcttcctat gaaatgtttt agagatctct tcaccatact ggtttcttct 3660
   ctttggttgg tgtgggtaaa agaaaacaaa acatttccta taagctgaaa gctgaccagc 3720
   attctcttct tggtaacatc tactactcca atctagaaaa tttggattct agaccaaaaa 3780
   tcaggaaaca tggctcctta taaatctgtg cagctgcctt atagtaccat caaaggaatt 3840
   tcaggtgggc tgggcggggc cccgatccca gaattatcaa ctccacccat catcatttgg 3900
   tcatgaagca tcctttcatt cttcttcttc tttttttggg gggggcgggg cggggagggg 3960
   atctcaaagt tttagtcttc cagaatccaa attaaaggtt gcccctgatg ggggccaggt 4020
   tccgccacag aacatcttag atgtcagcct tgacctcact tagcagggat tacagaaatg 4080
   agatacattt tgaaggagag ttgtctgtta tgttcactgt attctaagtg cctgggataa 4140
   agctgtctca tgggtgctcc atatatattc atatatattt gttgagtgaa ttaatgaatt 4200
   aagagtggct ggcagagtag gcagaaaaag acactgcaaa tggcataaaa attaaagtcc 4260
   tagctgagtt ctcaatggta aaggcatcag atgtcttagc agtcaagcta gaaattcatg 4320
   acaatgagta ttactatttg cctaatggca actcattgct ctccatgtaa atgtaatcaa 4380
   cagatgaaga gaatataatt gctctgcttt tccactaaaa ctccatctta gtgaatttta 4440
   aattatccag agatgtcaaa ctgccaaata aaaatatttc agtagtcttt gcatcagctt 4500
   accttgtacc agaaacattt ccaatttact atcaaattat agtaactgag cctgtgtgaa 4560
   gtatctcatc attttcgaaa ggaacacctt gtgtgatgcc agtgagcatt tctaaaaagg 4620
   gtgtgaggta gaggtaaaaa taaggtgaga gaccatttca gaatgcactg ttgctcaaaa 4680
   aggtgatctg gttctttctt cagagatttc tacggggata gaaaatcggg agtctgccct 4740
   cattaatctg tgattccacc tcttgcacca aatcaatatc tatttgttga gcacttattg 4800
   attaagacct tgcatatgtc tgtccatttt gatttgagat acaacttttt gtgtgggttg 4860
   aatgacaaat cactccaaac aaagctgggc acagagaatc agctaggaga ccagttattc 4920
   agggtccatt tctcttggat gtaaaggagt cctgggtaaa atgtggctgt aacctaaacc 4980
   aactagtcct tgtgatttgt ttctgccctc tgtgtttcct gttgtcaaat gctaagtgtg 5040
   tgttttgcag tcatgaacta aagcacaaaa agatgcatga gacattgtag tcatatgtct 5100
   ggtgtgacac tttggagcaa aaaccttgca gtggtaaata aaaaatttcc aacagggaaa 5160
   aaaaaaaaaa aaaaaaaaaa aaaa 5184

<210> 2
<211> 5388
<212> DNA
<213> Homo sapiens

<400> 2
gtccccgccc ctctggcggc gggagagctg ctggctcgcc cggatcccgg gagctgcctg 60

gaggcgggcc cggcccgggg aaggtgagcg gctgcgggac ccagcccctc gccgggagcg 120

ggcaccatgg tgctgtcggt gcctgtgatc gcgctgggcg ccacgctggg cacagccacc 180

agcatcctcg cgttgtgcgg ggtcacctgc ctgtgtcggc acatgcaccc caagaagggg 240

ctgctgccgc gggaccagga ccccgacctg gagaaggcga agcccagctt gctcgggtct 300

gcacaacaga gcatctgtta catatgaagt ttacagaggt ggtggccaaa gggctttgtg 360

atgtgactgc tgaaataatg caggggccac ctacaagaag gaaatctcta gcctaaaaaa 420

gcaactcaga atgtggagtg ggatagcctg caaatcactt gtcacttaag gtgacggccc 480

agagaggtga aatgacctgc ccaaagtgac ccagctccag cggcaggttc aatgttaaaa 540

agtccacgga acctgttcag ccccgtgccc tcctcaagtt cccagacatc tatggaccca 600

ggccagctgt gacggctcca gaggtcatca actatgcaga ctattcactg aggtctacgg 660

aggagcccac tgcacctgcc agcccccaac ccccgaatga cagtcgcctc aagaggcagg 720

tcacagagga gctgttcatc ctccctcaga atggtgtggt ggaggatgtc tgtgtcatgg 780

agacctggaa cccagagaag gctgccagtt ggaaccaggc ccccaaactc cactactgcc 840

tggactatga ctgtcagaag gcagaattgt ttgtgactcg cctggaagct gtgaccagca 900

accacgacgg aggctgtgac tgctacgtcc aagggagtgt ggccaatagg accggctctg 960

tggaggctca gacagcccta aagaagcggc agctgcacac cacctgggag gagggcctgg 1020

tgctccccct ggcggaggag gagctcccca cagccaccct gacgctgacc ttgaggacct 1080

gcgaccgctt ctcccgtcac agcgtggccg gggagctccg cctgggcctg gacgggacat 1140

ctgtgcctct aggggctgcc cagtggggcg agctgaagac ttcagcgaag gagccatctg 1200

caggagctgg agaggtccta ctatccatca gctacctccc ggctgccaac cgcctcctgg 1260

tggtgctgat taaagccaag aacctccact ctaaccagtc caaggagctc ctggggaagg 1320

atgtctctgt caaggtgacc ttgaagcacc aggctcggaa gctgaagaag aagcagacta 1380

aacgagctaa gcacaagatc aaccccgtgt ggaacgagat gatcatgttt gagctgcctg 1440

acgacctgct gcaggcctcc agtgtggagc tggaagtgct gggccaggac gattcagggc 1500

agagctgtgc gcttggccac tgcagcctgg gcctgcacac ctcgggctct gagcgcagcc 1560

actgggagga gatgctcaaa aaccctcgcc ggcagattgc catgtggcac cagctgcacc 1620

tgtaaccagc tgcccagctg cctcccttct tggacagccc tgacccgtcc tctgcaacct 1680

cctttctgtg cccctttcct cattctgaca cccagaagac agtgacagat gtgtttgcaa 1740

ggctgggatg gctctctcat catactcttg tttcttagaa ataagcaaga cagagcagga 1800

aatggaatat gcgggtcaca ctgaggaatg cattttgctc atctgtgtta ttgaaggagg 1860

tgcttattaa atacagttcc tatgcctgtt ttataggtgg ggttaggcca gatgcagaga 1920

aagctaaatg tgggaatcat ggatgcaaag aagaatttgg ctttttgaaa aacaagcatt 1980

tcaaaaatga tgaaggaagt gaaagtatcc tggatcaact cctagagtta gagattgccc 2040

aggtggaaag aaaccttagc cagcgttcaa tcaagctcac catgcagggc agtcacccgg 2100

cagttctcaa actttagcat gtgaagagtc accagcagat tcctgggctc gcctggagac 2160

attcctagtc ggtattcctg gtcgaagccc aggagccttc ctttttaaca agctgatgta 2220

gagggtggag cactgtatgt ggagaaattc cttctacaat attccacaca ggtttttggc 2280

cacagtcctt gatggagtcc caaaaccatg gtgcagccag ttccaatgct ggacacctca 2340

accatcaggg tgaaatctgg ggcctcagct ttttaattta attattttaa ttcttaatac 2400

tttaatttgt gcatttcata agccccctgc tcttggactg aattttgtgc tttttattga 2460

agaattttat tgtttttatc ttaaaatcag tttctattat ccttggggag accatcccta 2520

acaaagtaca ggtgggatct cctgtgagtc attggctggg ttctgattgc tagatgtcac 2580

acccaccagc atcaccaaag tgactctgag atagaccggt cccttctcag cgttccagtc 2640

acttcaggag gaatttagtt attgacttag tctatgacat ctggctacat gtaggtagag 2700

aagaaagata attttaaaaa ggaaatcagg tcttttgcaa ctgtgcctcc ctctgtctgt 2760

tttcacttga atgggtaaat aaccagcagc taggttttga attcctacct tgttattcta 2820

aacagatgtc cacattgtta attaaatcta aattatgagc cttgctgagt ggatacggta 2880

cttacacctg aaccaggatt cctgggttct gttgttgaca ttgcccttca gcacctgttt 2940

ggccagctgt gtaagatagg actaatgact aggaagccta ccccaatgaa tgatatacta 3000

gatgaaatag tgttcaaaac ctgtaggcac tctctggcta aaaacaaact ctgaggccac 3060

cagcagatca tctttaagct aagttactat ttttcacctt tttttttaga cggagttttg 3120

ctctttgttg cccaggctgg agtgcagtgg cacgatctcg gctcactgca acctccgcct 3180

cccaagttca agcgattctc ctctctcagc ctcctgggta gctgggatta caggtgccca 3240

ccaacatgcc tggctaattt ttgtactttt agtagagatg gggtttcacc atgttggcca 3300

ggctggtctt caactccaga tctcaggtga tctaccctcc tcggcctccc aaagtactgg 3360

gattacaggc ctgagccacc gcgcccggcc tatttttcac tttaatttgg cagctgagaa 3420

tgcccagaaa gtgccagaag catcgtggca tttccagaac catggattct gcctttggac 3480

ccctctctat taatattaaa actctgggcc ttcagatgtc accctaatcc acttccctaa 3540

gacagaattt ctggacaaga tgggtaaggg cttcattcct tcaacaagtc aagtcatact 3600

tggcctctcc ctgagaatct gagcaggagc cttataacct gtggtcatta ttttttcttt 3660

ctgtacagaa atagaaaagc attagaaata acttctaacc atcctctgaa aaaacagaaa 3720

aaatatcgaa tccctctttc atgagaagtc ttttggataa ttggaaacct tcatcactga 3780

ggttggccag cccctgccaa gtgttgtgta ggcaaagcac ttgttagtgg cttcctatga 3840

aatgttttag agatctcttc accatactgg tttcttctct ttggttggtg tgggtaaaag 3900

aaaacaaaac atttcctata agctgaaagc tgaccagcat tctcttcttg gtaacatcta 3960

ctactccaat ctagaaaatt tggattctag accaaaaatc aggaaacatg gctccttata 4020

aatctgtgca gctgccttat agtaccatca aaggaatttc aggtgggctg ggcggggccc 4080

cgatcccaga attatcaact ccacccatca tcatttggtc atgaagcatc ctttcattct 4140

tcttcttctt tttttggggg gggcggggcg ggggagggat ctcaaagttt tagtcttcca 4200

gaatccaaat taaaggttgc ccctgatggg ggccaggttc cgccacagaa catcttagat 4260

gtcagccttg acctcactta gcagggatta cagaaatgag atacattttg aaggagagtt 4320

gtctgttatg ttcactgtat tctaagtgcc tgggataaag ctgtctcatg ggtgctccat 4380

atatattcat atatatttgt tgagtgaatt aatgaattaa gagtggctgg cagagtaggc 4440

agaaaaagac actgcaaatg gcataaaaat taaagtccta gctgagttct caatggtaaa 4500

ggcatcagat gtcttagcag tcaagctaga aattcatgac aatgagtatt actatttgcc 4560

taatggcaac tcattgctct ccatgtaaat gtaatcaaca gatgaagaga atataattgc 4620

tctgcttttc cactaaaact ccatcttagt gaattttaaa ttatccagag atgtcaaact 4680

gccaaataaa aatatttcag tagtctttgc atcagcttac cttgtaccag aaacatttcc 4740

aatttactat caaattatag taactgagcc tgtgtgaagt atctcatcat tttcgaaagg 4800

aacaccttgt gtgatgccag tgagcatttc taaaaagggt gtgaggtaga ggtaaaaata 4860

aggtgagaga ccatttcaga atgcactgtt gctcaaaaag gtgatctggt tctttcttca 4920

gagatttcta cggggataga aaatcgggag tctgccctca ttaatctgtg attccacctc 4980

ttgcaccaaa tcaatatcta tttgttgagc acttattgat taagaccttg catatgtctg 5040

tccattttga tttgagatac aactttttgt gtgggttgaa tgacaaatca ctccaaacaa 5100

agctgggcac agagaatcag ctaggagacc agttattcag ggtccatttc tcttggatgt 5160

aaaggagtcc tgggtaaaat gtggctgtaa cctaaaccaa ctagtccttg tgatttgttt 5220

ctgccctctg tgtttcctgt tgtcaaatgc taagtgtgtg ttttgcagtc atgaactaaa 5280

gcacaaaaag atgcatgaga cattgtagtc atatgtctgg tgtgacactt tggagcaaaa 5340

accttgcagt ggtaaataaa aaatttccaa cagggaaaaa aaaaaaaa 5388

<210> 3
<211> 15
<212> DNA
<213> Artificial

<220>
<223> hSYT13-350-AmNA(15)

<220>
<221> misc_feature
<222> (1)..(3), (13)..(14)
<223> AmNA

<400> 3
ggaacttgag gaggg 15

<210> 4
<211> 15
<212> DNA
<213> Artificial

<220>
<223> hSYT13-605-AmNA(15)

<220>
<221> misc_feature
<222> (1)..(3), (13)..(14)
<223> AmNA

<400> 4
agtagtggag tttgg 15

<210> 5
<211> 15
<212> DNA
<213> Artificial

<220>
<223> hSYT13-999-AmNA(15)

<220>
<221> misc_feature
<222> (1)..(1)
<223> 5mC

<220>
<221> misc_feature
<222> (1)..(3), (13)..(14)
<223> AmNA

<400> 5
ctgatggata gtagg 15

<210>  6
<211>  15
<212>  DNA
<213>  Artificial

<220>
<223>  hSYT13-1000-AmNA(15)

<220>
<221>  misc_feature
<222>  (2)..(2)
<223>  5mC

<220>
<221>  misc_feature
<222>  (1)..(3), (13)..(14)
<223>  AmNA

<400>  6
gctgatggat agtag                                                        15


<210>  7
<211>  15
<212>  DNA
<213>  Artificial

<220>
<223>  hSYT13-1071-AmNA(15)

<220>
<221>  misc_feature
<222>  (1)..(3), (13)..(14)
<223>  AmNA

<400>  7
ttggactggt tagag                                                        15


<210>  8
<211>  15
<212>  DNA
<213>  Artificial

<220>
<223>  hSYT13-1072-AmNA(15)

<220>
<221>  misc_feature
<222>  (1)..(1)
<223>  5mC

<220>
<221>  misc_feature
<222>  (1)..(3), (13)..(14)
<223>  AmNA

<400>  8
cttggactgg ttaga                                                        15


<210>  9

<211>  15
<212>  DNA
<213>  Artificial

<220>
<223>  hSYT13-1421-AmNA(15)

<220>
<221>  misc_feature
<222>  (1)..(2), (14)..(14)
<223>  5mC

<220>
<221>  misc_feature
<222>  (1)..(3), (13)..(14)
<223>  AmNA

<400>  9
ccaagaaggg aggca                                                        15


<210>  10
<211>  15
<212>  DNA
<213>  Artificial

<220>
<223>  hSYT13-1614-AmNA(15)

<220>
<221>  misc_feature
<222>  (2)..(2)
<223>  5mC

<220>
<221>  misc_feature
<222>  (1)..(3), (13)..(14)
<223>  AmNA

<400>  10
acagatgagc aaaat                                                        15


<210>  11
<211>  15
<212>  DNA
<213>  Artificial

<220>
<223>  hSYT13-1617-AmNA(15)

<220>
<221>  misc_feature
<222>  (3)..(3), (13)..(13)
<223>  5mC

<220>
<221>  misc_feature
<222>  (1)..(3), (13)..(14)
<223>  AmNA

<400>  11
aacacagatg agcaa                                                        15

<210>  12
<211>  15
<212>  DNA
<213>  Artificial

<220>
<223>  hSYT13-1618-AmNA(15)

<220>
<221>  misc_feature
<222>  (14)..(14)
<223>  5mC

<220>
<221>  misc_feature
<222>  (1)..(3), (13)..(14)
<223>  AmNA

<400>  12
taacacagat gagca                                                        15


<210>  13
<211>  15
<212>  DNA
<213>  Artificial

<220>
<223>  hSYT13-1619-AmNA(15)

<220>
<221>  misc_feature
<222>  (1)..(3), (13)..(14)
<223>  AmNA

<400>  13
ataacacaga tgagc                                                        15


<210>  14
<211>  15
<212>  DNA
<213>  Artificial

<220>
<223>  hSYT13-1622-AmNA(15)

<220>
<221>  misc_feature
<222>  (2)..(2)
<223>  5mC

<220>
<221>  misc_feature
<222>  (1)..(3), (13)..(14)
<223>  AmNA

<400>  14
tcaataacac agatg                                                        15

<210>  15
<211>  15
<212>  DNA
<213>  Artificial

<220>
<223>  hSYT13-1623-AmNA(15)

<220>
<221>  misc_feature
<222>  (3)..(3)
<223>  5mC

<220>
<221>  misc_feature
<222>  (1)..(3), (13)..(14)
<223>  AmNA

<400>  15
ttcaataaca cagat                                                        15


<210>  16
<211>  15
<212>  DNA
<213>  Artificial

<220>
<223>  hSYT13-1625-AmNA(15)

<220>
<221>  misc_feature
<222>  (1)..(2), (13)..(13)
<223>  5mC

<220>
<221>  misc_feature
<222>  (1)..(3), (13)..(14)
<223>  AmNA

<400>  16
ccttcaataa cacag                                                        15


<210>  17
<211>  15
<212>  DNA
<213>  Artificial

<220>
<223>  hSYT13-1777-AmNA(15)

<220>
<221>  misc_feature
<222>  (14)..(14)
<223>  5mC

<220>
<221>  misc_feature
<222>  (1)..(3), (13)..(14)
<223>  AmNA

<400>  17

gatactttca cttcc                                                    15


<210>  18
<211>  15
<212>  DNA
<213>  Artificial

<220>
<223>  hSYT13-2631-AmNA(15)

<220>
<221>  misc_feature
<222>  (1)..(3), (13)..(14)
<223>  AmNA

<400>  18
aaggctcata attta                                                    15


<210>  19
<211>  15
<212>  DNA
<213>  Artificial

<220>
<223>  hSYT13-2812-AmNA(15)

<220>
<221>  misc_feature
<222>  (1)..(3), (13)..(14)
<223>  AmNA

<400>  19
tttttagcca gagag                                                    15


<210>  20
<211>  15
<212>  DNA
<213>  Artificial

<220>
<223>  hSYT13-2813-AmNA(15)

<220>
<221>  misc_feature
<222>  (1)..(3), (13)..(14)
<223>  AmNA

<400>  20
gtttttagcc agaga                                                    15


<210>  21
<211>  15
<212>  DNA
<213>  Artificial

<220>
<223>  hSYT13-2814-AmNA(15)

<220>

<221>  misc_feature
<222>  (1)..(3), (13)..(14)
<223>  AmNA

<400>  21
tgtttttagc cagag                                                        15


<210>  22
<211>  15
<212>  DNA
<213>  Artificial

<220>
<223>  hSYT13-2815-AmNA(15)

<220>
<221>  misc_feature
<222>  (1)..(3), (13)..(14)
<223>  AmNA

<400>  22
ttgtttttag ccaga                                                        15


<210>  23
<211>  15
<212>  DNA
<213>  Artificial

<220>
<223>  hSYT13-3246-AmNA(15)

<220>
<221>  misc_feature
<222>  (1)..(2), (14)..(14)
<223>  5mC

<220>
<221>  misc_feature
<222>  (1)..(3), (13)..(14)
<223>  AmNA

<400>  23
ccaaaggcag aatcc                                                        15


<210>  24
<211>  15
<212>  DNA
<213>  Artificial

<220>
<223>  hSYT13-3317-AmNA(15)

<220>
<221>  misc_feature
<222>  (1)..(3), (13)..(14)
<223>  AmNA

<400>  24
aattctgtct taggg                                                        15

<210>  25
<211>  15
<212>  DNA
<213>  Artificial

<220>
<223>  hSYT13-3425-AmNA(15)

<220>
<221>  misc_feature
<222>  (14)..(14)
<223>  5mC

<220>
<221>  misc_feature
<222>  (1)..(3), (13)..(14)
<223>  AmNA

<400>  25
gaaaaaataa tgacc                                                        15


<210>  26
<211>  15
<212>  DNA
<213>  Artificial

<220>
<223>  hSYT13-3426-AmNA(15)

<220>
<221>  misc_feature
<222>  (1)..(3), (13)..(14)
<223>  AmNA

<400>  26
agaaaaaata atgac                                                        15


<210>  27
<211>  15
<212>  DNA
<213>  Artificial

<220>
<223>  hSYT13-4268-AmNA(15)

<220>
<221>  misc_feature
<222>  (1)..(3), (13)..(14)
<223>  AmNA

<400>  27
tttaccattg agaac                                                        15


<210>  28
<211>  15
<212>  DNA
<213>  Artificial

<220>

<223>  hSYT13-4330-AmNA(15)

<220>
<221>  misc_feature
<222>  (1)..(3), (13)..(14)
<223>  AmNA

<400>  28
taggcaaata gtaat                                                        15


<210>  29
<211>  15
<212>  DNA
<213>  Artificial

<220>
<223>  hSYT13-4367-AmNA(15)

<220>
<221>  misc_feature
<222>  (1)..(3), (13)..(14)
<223>  AmNA

<400>  29
gttgattaca tttac                                                        15


<210>  30
<211>  15
<212>  DNA
<213>  Artificial

<220>
<223>  hSYT13-4368-AmNA(15)

<220>
<221>  misc_feature
<222>  (1)..(3), (13)..(14)
<223>  AmNA

<400>  30
tgttgattac attta                                                        15


<210>  31
<211>  15
<212>  DNA
<213>  Artificial

<220>
<223>  hSYT13-4371-AmNA(15)

<220>
<221>  misc_feature
<222>  (3)..(3), (13)..(13)
<223>  5mC

<220>
<221>  misc_feature
<222>  (1)..(3), (13)..(14)
<223>  AmNA

<400>  31
atctgttgat tacat                                                        15


<210>  32
<211>  15
<212>  DNA
<213>  Artificial

<220>
<223>  hSYT13-4373-AmNA(15)

<220>
<221>  misc_feature
<222>  (2)..(2)
<223>  5mC

<220>
<221>  misc_feature
<222>  (1)..(3), (13)..(14)
<223>  AmNA

<400>  32
tcatctgttg attac                                                        15


<210>  33
<211>  15
<212>  DNA
<213>  Artificial

<220>
<223>  hSYT13-4374-AmNA(15)

<220>
<221>  misc_feature
<222>  (3)..(3)
<223>  5mC

<220>
<221>  misc_feature
<222>  (1)..(3), (13)..(14)
<223>  AmNA

<400>  33
ttcatctgtt gatta                                                        15


<210>  34
<211>  15
<212>  DNA
<213>  Artificial

<220>
<223>  hSYT13-4377-AmNA(15)

<220>
<221>  misc_feature
<222>  (1)..(1), (3)..(3)
<223>  5mC

<220>
<221>  misc_feature

<222>  (1)..(3), (13)..(14)
<223>  AmNA

<400>  34
ctcttcatct gttga                                                        15


<210>  35
<211>  15
<212>  DNA
<213>  Artificial

<220>
<223>  hSYT13-4378-AmNA(15)

<220>
<221>  misc_feature
<222>  (2)..(2)
<223>  5mC

<220>
<221>  misc_feature
<222>  (1)..(3), (13)..(14)
<223>  AmNA

<400>  35
tctcttcatc tgttg                                                        15


<210>  36
<211>  15
<212>  DNA
<213>  Artificial

<220>
<223>  hSYT13-4381-AmNA(15)

<220>
<221>  misc_feature
<222>  (13)..(13)
<223>  5mC

<220>
<221>  misc_feature
<222>  (1)..(3), (13)..(14)
<223>  AmNA

<400>  36
tattctcttc atctg                                                        15


<210>  37
<211>  15
<212>  DNA
<213>  Artificial

<220>
<223>  hSYT13-4716-AmNA(15)

<220>
<221>  misc_feature
<222>  (1)..(3), (14)..(14)
<223>  5mC

<220>
<221>  misc_feature
<222>  (1)..(3), (13)..(14)
<223>  AmNA

<400>  37
   cccgattttc tatcc                                                          15


<210>  38
<211>  15
<212>  DNA
<213>  Artificial

<220>
<223>  hSYT13-4717-AmNA(15)

<220>
<221>  misc_feature
<222>  (2)..(3)
<223>  5mC

<220>
<221>  misc_feature
<222>  (1)..(3), (13)..(14)
<223>  AmNA

<400>  38
tcccgatttt ctatc                                                         15


<210>  39
<211>  15
<212>  DNA
<213>  Artificial

<220>
<223>  hSYT13-4729-AmNA(15)

<220>
<221>  misc_feature
<222>  (14)..(14)
<223>  5mC

<220>
<221>  misc_feature
<222>  (1)..(3), (13)..(14)
<223>  AmNA

<400>  39
atgagggcag actcc                                                         15


<210>  40
<211>  15
<212>  DNA
<213>  Artificial

<220>
<223>  hSYT13-4778-AmNA(15)

<220>

<221>  misc_feature
<222>  (2)..(2)
<223>  5mC

<220>
<221>  misc_feature
<222>  (1)..(3), (13)..(14)
<223>  AmNA

<400>  40
gctcaacaaa tagat                                                        15


<210>  41
<211>  15
<212>  DNA
<213>  Artificial

<220>
<223>  hSYT13-4779-AmNA(15)

<220>
<221>  misc_feature
<222>  (3)..(3)
<223>  5mC

<220>
<221>  misc_feature
<222>  (1)..(3), (13)..(14)
<223>  AmNA

<400>  41
tgctcaacaa ataga                                                        15


<210>  42
<211>  15
<212>  DNA
<213>  Artificial

<220>
<223>  hSYT13-4951-AmNA(15)

<220>
<221>  misc_feature
<222>  (1)..(1), (3)..(3)
<223>  5mC

<220>
<221>  misc_feature
<222>  (1)..(3), (13)..(14)
<223>  AmNA

<400>  42
cacattttac ccagg                                                        15


<210>  43
<211>  15
<212>  DNA
<213>  Artificial

<220>

<223>  hSYT13-4952-AmNA(15)

<220>
<221>  misc_feature
<222>  (1)..(2), (13)..(13)
<223>  5mC

<220>
<221>  misc_feature
<222>  (1)..(3), (13)..(14)
<223>  AmNA

<400>  43
ccacatttta cccag                                                        15


<210>  44
<211>  15
<212>  DNA
<213>  Artificial

<220>
<223>  NEG1

<220>
<221>  misc_feature
<222>  (1)..(1), (3)..(3)
<223>  5mC

<220>
<221>  misc_feature
<222>  (1)..(3), (13)..(14)
<223>  AmNA

<400>  44
cacagtatct atgta                                                        15


<210>  45
<211>  15
<212>  DNA
<213>  Artificial

<220>
<223>  NEG2

<220>
<221>  misc_feature
<222>  (1)..(3), (13)..(14)
<223>  AmNA

<400>  45
gaatctcaca gtatc                                                        15


<210>  46
<211>  19
<212>  DNA
<213>  Artificial

<220>
<223>  hGAPDH-Forward primer

<400>  46
cgacagtcag ccgcatctt                                                    19


<210>  47
<211>  22
<212>  DNA
<213>  Artificial

<220>
<223>  hGAPDH-Reverse primer

<400>  47
cccaatacga ccaaatccgt tg                                                22


<210>  48
<211>  20
<212>  DNA
<213>  Artificial

<220>
<223>  hSYT13-Forward primer

<400>  48
tggtggtgct gattaaagcc                                                   20


<210>  49
<211>  20
<212>  DNA
<213>  Artificial

<220>
<223>  hSYT13-Reverse primer

<400>  49
tgcttcttct tcagcttccg                                                   20


<210>  50
<211>  13
<212>  DNA
<213>  Artificial

<220>
<223>  hSYT13-607-AmNA(13)

<220>
<221>  misc_feature
<222>  (1)..(3), (11)..(12)
<223>  AmNA

<400>  50
agtagtggag ttt                                                          13


<210>  51
<211>  13
<212>  DNA
<213>  Artificial

<220>

<223>  hSYT13-609-AmNA(13)

<220>
<221>  misc_feature
<222>  (2)..(2)
<223>  5mC

<220>
<221>  misc_feature
<222>  (1)..(3), (11)..(12)
<223>  AmNA

<400>  51
gcagtagtgg agt                                                          13


<210>  52
<211>  15
<212>  DNA
<213>  Artificial

<220>
<223>  hSYT13-603-AmNA(15)

<220>
<221>  misc_feature
<222>  (1)..(3), (13)..(14)
<223>  AmNA

<400>  52
tagtggagtt tgggg                                                        15


<210>  53
<211>  15
<212>  DNA
<213>  Artificial

<220>
<223>  hSYT13-607-AmNA(15)

<220>
<221>  misc_feature
<222>  (2)..(2)
<223>  5mC

<220>
<221>  misc_feature
<222>  (1)..(3), (13)..(14)
<223>  AmNA

<400>  53
gcagtagtgg agttt                                                        15


<210>  54
<211>  15
<212>  DNA
<213>  Artificial

<220>
<223>  hSYT13-609-AmNA(15)

<220>
<221>  misc_feature
<222>  (1)..(3), (13)..(14)
<223>  AmNA

<400>  54
aggcagtagt ggagt                                                        15


<210>  55
<211>  17
<212>  DNA
<213>  Artificial

<220>
<223>  hSYT13-601-AmNA(17)

<220>
<221>  misc_feature
<222>  (1)..(3), (15)..(16)
<223>  AmNA

<400>  55
tagtggagtt tgggggc                                                      17


<210>  56
<211>  17
<212>  DNA
<213>  Artificial

<220>
<223>  hSYT13-603-AmNA(17)

<220>
<221>  misc_feature
<222>  (1)..(3), (15)..(16)
<223>  AmNA

<400>  56
agtagtggag tttgggg                                                      17


<210>  57
<211>  17
<212>  DNA
<213>  Artificial

<220>
<223>  hSYT13-605-AmNA(17)

<220>
<221>  misc_feature
<222>  (2)..(2)
<223>  5mC

<220>
<221>  misc_feature
<222>  (1)..(3), (15)..(16)
<223>  AmNA

<400>  57
gcagtagtgg agtttgg                                                      17

<210>  58
<211>  17
<212>  DNA
<213>  Artificial

<220>
<223>  hSYT13-607-AmNA(17)

<220>
<221>  misc_feature
<222>  (1)..(3), (15)..(16)
<223>  AmNA

<400>  58
aggcagtagt ggagttt                                                    17


<210>  59
<211>  17
<212>  DNA
<213>  Artificial

<220>
<223>  hSYT13-609-AmNA(17)

<220>
<221>  misc_feature
<222>  (1)..(2)
<223>  5mC

<220>
<221>  misc_feature
<222>  (1)..(3), (15)..(16)
<223>  AmNA

<400>  59
ccaggcagta gtggagt                                                    17


<210>  60
<211>  13
<212>  DNA
<213>  Artificial

<220>
<223>  hSYT13-4374-AmNA(13)

<220>
<221>  misc_feature
<222>  (1)..(1)
<223>  5mC

<220>
<221>  misc_feature
<222>  (1)..(3), (11)..(12)
<223>  AmNA

<400>  60
catctgttga tta                                                        13

<210>  61
<211>  15
<212>  DNA
<213>  Artificial

<220>
<223>  hSYT13-4376-AmNA(15)

<220>
<221>  misc_feature
<222>  (2)..(2)
<223>  5mC

<220>
<221>  misc_feature
<222>  (1)..(3), (13)..(14)
<223>  AmNA

<400>  61
tcttcatctg ttgat                                                        15


<210>  62
<211>  15
<212>  DNA
<213>  Artificial

<220>
<223>  hSYT13-4380-AmNA(15)

<220>
<221>  misc_feature
<222>  (1)..(3), (13)..(14)
<223>  AmNA

<400>  62
attctcttca tctgt                                                        15


<210>  63
<211>  15
<212>  DNA
<213>  Artificial

<220>
<223>  hSYT13-4382-AmNA(15)

<220>
<221>  misc_feature
<222>  (14)..(14)
<223>  5mC

<220>
<221>  misc_feature
<222>  (1)..(3), (13)..(14)
<223>  AmNA

<400>  63
atattctctt catct                                                        15


<210>  64
<211>  17

<212>  DNA
<213>  Artificial

<220>
<223>  hSYT13-4374-AmNA(17)

<220>
<221>  misc_feature
<222>  (2)..(2)
<223>  5mC

<220>
<221>  misc_feature
<222>  (1)..(3), (15)..(16)
<223>  AmNA

<400>  64
tcttcatctg ttgatta                                                    17


<210>  65
<211>  17
<212>  DNA
<213>  Artificial

<220>
<223>  hSYT13-4376-AmNA(17)

<220>
<221>  misc_feature
<222>  (2)..(2)
<223>  5mC

<220>
<221>  misc_feature
<222>  (1)..(3), (15)..(16)
<223>  AmNA

<400>  65
tctcttcatc tgttgat                                                    17


<210>  66
<211>  17
<212>  DNA
<213>  Artificial

<220>
<223>  hSYT13-4378-AmNA(17)

<220>
<221>  misc_feature
<222>  (1)..(3), (15)..(16)
<223>  AmNA

<400>  66
attctcttca tctgttg                                                    17


<210>  67
<211>  17
<212>  DNA
<213>  Artificial

<220>
<223>  hSYT13-4380-AmNA(17)

<220>
<221>  misc_feature
<222>  (1)..(3), (15)..(16)
<223>  AmNA

<400>  67
atattctctt catctgt                                                    17


<210>  68
<211>  17
<212>  DNA
<213>  Artificial

<220>
<223>  hSYT13-4382-AmNA(17)

<220>
<221>  misc_feature
<222>  (16)..(16)
<223>  5mC

<220>
<221>  misc_feature
<222>  (1)..(3), (15)..(16)
<223>  AmNA

<400>  68
ttatattctc ttcatct                                                    17


<210>  69
<211>  19
<212>  DNA
<213>  Artificial

<220>
<223>  hSYT13-4374-AmNA(19)

<220>
<221>  misc_feature
<222>  (2)..(2)
<223>  5mC

<220>
<221>  misc_feature
<222>  (1)..(3), (17)..(18)
<223>  AmNA

<400>  69
tctcttcatc tgttgatta                                                  19


<210>  70
<211>  13
<212>  DNA
<213>  Artificial

<220>

<223>  hSYT13-4725-AmNA(13)

<220>
<221>  misc_feature
<222>  (2)..(2)
<223>  5mC

<220>
<221>  misc_feature
<222>  (1)..(3), (11)..(12)
<223>  AmNA

<400>  70
gcagactccc gat                                                          13


<210>  71
<211>  13
<212>  DNA
<213>  Artificial

<220>
<223>  hSYT13-4727-AmNA(13)

<220>
<221>  misc_feature
<222>  (11)..(12)
<223>  5mC

<220>
<221>  misc_feature
<222>  (1)..(3), (11)..(12)
<223>  AmNA

<400>  71
gggcagactc ccg                                                          13


<210>  72
<211>  15
<212>  DNA
<213>  Artificial

<220>
<223>  hSYT13-4725-AmNA(15)

<220>
<221>  misc_feature
<222>  (1)..(3), (13)..(14)
<223>  AmNA

<400>  72
gggcagactc ccgat                                                        15


<210>  73
<211>  15
<212>  DNA
<213>  Artificial

<220>
<223>  hSYT13-4727-AmNA(15)

<220>
<221>  misc_feature
<222>  (13)..(14)
<223>  5mC

<220>
<221>  misc_feature
<222>  (1)..(3), (13)..(14)
<223>  AmNA

<400>  73
gagggcagac tcccg                                                        15


<210>  74
<211>  15
<212>  DNA
<213>  Artificial

<220>
<223>  hSYT13-4731-AmNA(15)

<220>
<221>  misc_feature
<222>  (14)..(14)
<223>  5mC

<220>
<221>  misc_feature
<222>  (1)..(3), (13)..(14)
<223>  AmNA

<400>  74
taatgagggc agact                                                        15


<210>  75
<211>  17
<212>  DNA
<213>  Artificial

<220>
<223>  hSYT13-4725-AmNA(17)

<220>
<221>  misc_feature
<222>  (1)..(3), (15)..(16)
<223>  AmNA

<400>  75
gagggcagac tcccgat                                                      17


<210>  76
<211>  17
<212>  DNA
<213>  Artificial

<220>
<223>  hSYT13-4727-AmNA(17)

<220>
<221>  misc_feature

<222>  (15)..(16)
<223>  5mC

<220>
<221>  misc_feature
<222>  (1)..(3), (15)..(16)
<223>  AmNA

<400>  76
atgagggcag actcccg                                                    17


<210>  77
<211>  17
<212>  DNA
<213>  Artificial

<220>
<223>  hSYT13-4729-AmNA(17)

<220>
<221>  misc_feature
<222>  (16)..(16)
<223>  5mC

<220>
<221>  misc_feature
<222>  (1)..(3), (15)..(16)
<223>  AmNA

<400>  77
taatgagggc agactcc                                                    17


<210>  78
<211>  17
<212>  DNA
<213>  Artificial

<220>
<223>  hSYT13-4731-AmNA(17)

<220>
<221>  misc_feature
<222>  (16)..(16)
<223>  5mC

<220>
<221>  misc_feature
<222>  (1)..(3), (15)..(16)
<223>  AmNA

<400>  78
attaatgagg gcagact                                                    17


<210>  79
<211>  17
<212>  DNA
<213>  Artificial

<220>
<223>  hSYT13-4733-AmNA(17)

<220>
<221>  misc_feature
<222>  (1)..(3), (15)..(16)
<223>  AmNA

<400>  79
agattaatga gggcaga                                                    17


<210>  80
<211>  17
<212>  DNA
<213>  Artificial

<220>
<223>  4733-A

<220>
<221>  misc_feature
<222>  (1)..(3), (15)..(16)
<223>  AmNA

<400>  80
agattaatga gggcaga                                                    17


<210>  81
<211>  17
<212>  DNA
<213>  Artificial

<220>
<223>  4733-B

<220>
<221>  misc_feature
<222>  (1)..(3), (15)..(16)
<223>  AmNA

<400>  81
agattaatga gggcaga                                                    17


<210>  82
<211>  17
<212>  DNA
<213>  Artificial

<220>
<223>  4733-C

<220>
<221>  misc_feature
<222>  (1)..(3), (15)..(16)
<223>  AmNA

<400>  82
agattaatga gggcaga                                                    17


<210>  83
<211>  17

<212>  DNA
<213>  Artificial

<220>
<223>  4733-D

<220>
<221>  misc_feature
<222>  (1)..(3), (15)..(16)
<223>  AmNA

<400>  83
agattaatga gggcaga                                                    17


<210>  84
<211>  17
<212>  DNA
<213>  Artificial

<220>
<223>  4733-E

<220>
<221>  misc_feature
<222>  (1)..(3), (15)..(16)
<223>  AmNA

<400>  84
agattaatga gggcaga                                                    17


<210>  85
<211>  17
<212>  DNA
<213>  Artificial

<220>
<223>  4733-F

<220>
<221>  misc_feature
<222>  (1)..(3), (15)..(16)
<223>  AmNA

<400>  85
agattaatga gggcaga                                                    17


<210>  86
<211>  17
<212>  DNA
<213>  Artificial

<220>
<223>  4733-G

<220>
<221>  misc_feature
<222>  (1), (3), (15)..(16)
<223>  AmNA

<400>  86

agattaatga gggcaga                                                    17

<210>  87
<211>  17
<212>  DNA
<213>  Artificial

<220>
<223>  4733-H

<220>
<221>  misc_feature
<222>  (14)..(14)
<223>  5mC

<220>
<221>  misc_feature
<222>  (1), (3), (14), (16)
<223>  AmNA

<400>  87
agattaatga gggcaga                                                    17

<210>  88
<211>  17
<212>  DNA
<213>  Artificial

<220>
<223>  4733-I

<220>
<221>  misc_feature
<222>  (1), (3), (16)
<223>  AmNA

<400>  88
agattaatga gggcaga                                                    17

<210>  89
<211>  17
<212>  DNA
<213>  Artificial

<220>
<223>  4733-J

<220>
<221>  misc_feature
<222>  (1), (4), (15)..(16)
<223>  AmNA

<400>  89
agattaatga gggcaga                                                    17

<210>  90
<211>  17
<212>  DNA
<213>  Artificial

<220>
<223>  4733-K

<220>
<221>  misc_feature
<222>  (14)..(14)
<223>  5mC

<220>
<221>  misc_feature
<222>  (1), (4), (14), (16)
<223>  AmNA

<400>  90
agattaatga gggcaga                                                    17


<210>  91
<211>  17
<212>  DNA
<213>  Artificial

<220>
<223>  4733-L

<220>
<221>  misc_feature
<222>  (1), (4), (16)
<223>  AmNA

<400>  91
agattaatga gggcaga                                                    17


<210>  92
<211>  17
<212>  DNA
<213>  Artificial

<220>
<223>  4733-M

<220>
<221>  misc_feature
<222>  (14)..(14)
<223>  5mC

<220>
<221>  misc_feature
<222>  (1)..(3), (14), (16)
<223>  AmNA

<400>  92
agattaatga gggcaga                                                    17


<210>  93
<211>  17
<212>  DNA
<213>  Artificial

<220>

<223>  4733-N

<220>
<221>  misc_feature
<222>  (1)..(3), (16)
<223>  AmNA

<400>  93
agattaatga gggcaga                                                    17